(19) Europäisches Patentamt
European Patent Office
Office européen des brevets

(11) Publication number: **0 395 666 B1**

(12) # EUROPEAN PATENT SPECIFICATION

(45) Date of publication of patent specification: **04.05.94**

(51) Int. Cl.5: **C07C 255/50**, C07C 255/54, C09K 19/12, C09K 19/42, C09K 19/44, C09K 19/46, G02F 1/13

(21) Application number: **88909109.6**

(22) Date of filing: **19.10.88**

(86) International application number: **PCT/GB88/00880**

(87) International publication number: **WO 89/03821 (05.05.89 89/10)**

(54) **LATERALLY CYANO- AND FLUORO-SUBSTITUTED TERPHENYLS.**

(30) Priority: **19.10.87 GB 8724458**
**13.04.88 GB 8808677**

(43) Date of publication of application:
**07.11.90 Bulletin 90/45**

(45) Publication of the grant of the patent:
**04.05.94 Bulletin 94/18**

(84) Designated Contracting States:
**AT BE CH DE FR GB IT LI NL SE**

(56) References cited:
**EP-A- 0 132 377**
**DE-C- 3 216 281**
**US-A- 4 632 515**

(73) Proprietor: **The Secretary of State for Defence in Her Britannic Majesty's Government of the United Kingdom of Great Britain and Northern Ireland**
**Whitehall**
**London SW1A 2HB(GB)**

(72) Inventor: **GRAY, George William**
**Glenwood**
**Newgate Street**
**Cottingham**
**North Humberside HU16 4DZ(GB)**
Inventor: **LACEY, David**
**19 Kildale Close**
**Hull**
**North Humberside HU18 9NN(GB)**
Inventor: **HIRD, Michael**
**222 Anlaby Park Road South**
**Hull**
**North Humberside HU4 7BZ(GB)**
Inventor: **TOYNE, Kenneth Johnson**
**25 Hall Road**
**Hull**
**North Humberside HU6 8OW(GB)**

(74) Representative: **Beckham, Robert William et al**
**Defence Research Agency**
**Intellectual Property Department**
**DRA Farnborough**
**Farnborough, Hants. GU14 6TD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 395 666 B1

## Description

This invention relates to laterally cyano- substittuted terphenyls, in particular to those which may be used as constituents of liquid crystal mixtures. The invention also relates to such mixtures containing these terphenyls and to electro-optic devices which use them.

Liquid crystal materials are well known, and are commonly used in electro-optical devices such as watches, calculators, displays etc. Such materials are of two general types. There are those which use the electro-optical properties of the nematic (N) phase, such as the electrically controlled birefringence (ECB) effect, as described in M.F. Schieckel and K.Fahrenshon, "Defomation of nematic liquid crystals with vertical orientation in electrical fields". Appl Phys Lett (1971), 19, 3912. There are also those which use the electro-optical properties manifested by smectic phases. Examples of the latter include the ferroelectric effect manifested by certain chiral tilted smectic phases, see for example N A Clark and S T Lagerwall Appl-Phys Lett (1980) 36, 899 which offers the advantages of high speed and bistable properties. The chiral smectic C, F and I phases ($S_C^*$ $S_F^*$ and $S_I^*$ ) (the asterisk denoting chirality)are generally most favoured for such use, the $S_C^*$ being preferred because of its lower viscosity. Another electro-optical effect in smectic phases is the fast-switching electroclinic effect manifested by smectic A phases ($S_A$).

Nematic and ferroelectric smectic liquid crystal materials have a number of desirable requirements in common, which for example include ease of preparation, chemical and photochemical stability, low viscosity and a broad temperature range over which the useful phases (eg N, $S_C$, $S_F$ or $S_I$) persist. Such materials are generally mixtures of compounds and these requirements are sought in compounds or mixtures thereof intended for use in the materials.

Materials and compounds for use in ECB devices and $S_C^*$ devices have a number of specific desirable requirements. For example for use in ECB devices, compounds and materials should preferably show a high value for the ratio of elastic constants $K_3/K_1$, high values for the optical anisotropy $\Delta n$ and a negative dielectric anisotropy $\Delta E$. In devices which use the ferroelectric effect in $S_C^*$ materials it is desirable that compounds and mixtures show an $S_A$ phase at a temperature above the range over which the $S_C^*$ phase persists, and that undesirable phases such as $S_B$ do not appear.

A number of compounds are known which show broad $S_C$ phases, among which are the alkyl- or alkoxy- terminated fluoroterphenyls ("FTP's") described in EP-A-0132377, of general formula:

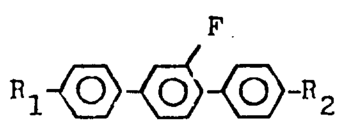

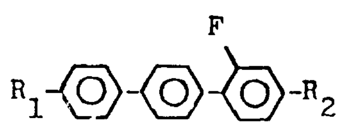

where $R_1$ and $R_2$ are alkyl or alkoxy. Some FTPs and mixtures thereof are known to show broad temperature range $S_C$ phases. This has made them excellent candidates for constituents of ferroelectric smectic mixtures. See for example their use in the mixtures disclosed in PCT/GB 87/0441 and GB 8627107.

It is an object of the present invention to provide novel compounds which may be used as constituents of liquid crystal mixtures, and novel liquid crystal mixtures which incorporate them.

According to this invention novel laterally cyano-substituted terphenyls of general formula I below are provided:

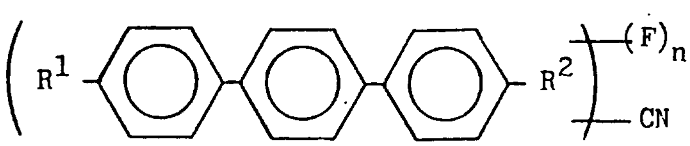

I

wherein $R^1$ and $R^2$ are independently selected from hydrogen or $C_{1-15}$ alkyl, alkoxy, or alkyl or alkoxy in which one or more $CH_2$ groups are replaced by O, COO, OOC, CHX, $CX_2$, CH=CX, CX=CH, CX=CX, where X is fluorine or chlorine, CRCN where R is alkyl, or C≡C, or in which a terminal $CH_3$ of the said alkyl or alkoxy chain is replaced by $CF_3$, n is 0 or 1, and the CN and F (if present) substituent are independently

located in any of the available substitution positions.

The structural preferences discussed below are based on inter alia on ease of preparation and suitability for use as constituents of liquid crystal mixtures and electro-optical devices which use them.

Preferred structures for terphenyls of formula I are those having a general formula IA below:

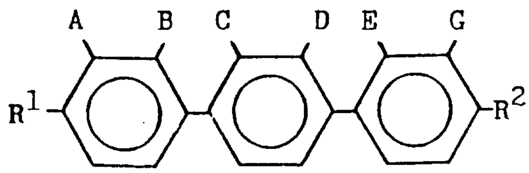

IA

wherein $R^1$ and $R^2$ are independently alkyl, alkoxy or alkynyl, and the terphenyl has a substitution pattern selected from any one of the following substitution patterns: (D = CN), (A = CN), (B = F, G = CN), (A = CN, B = F), (B = CN), (C = CN, D = F), (A = F, G = CN), (A = F, E = CN), (B = F, E = CN), (B = F, D = CN), (A = F, D = CN), (A = F, C = CN), ( B = F, C = CN), ( A = F, B = CN ) the remaining lateral substitution positions being occupied by hydrogen.

The structures included in formula IA are listed below in Table 1.

Table 1

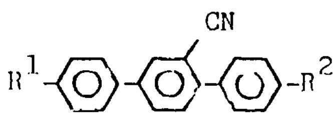

1.1

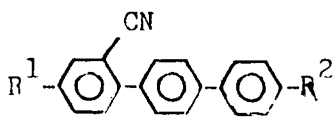

1.2

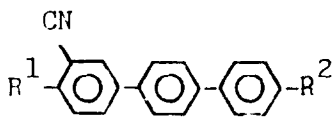

1.3

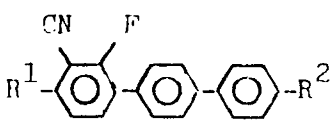

1.4

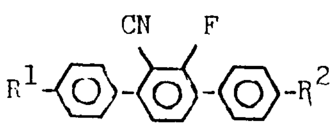

1.5

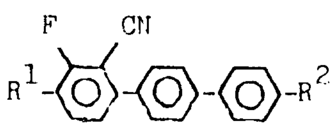

1.6

Table 1 (contd.)

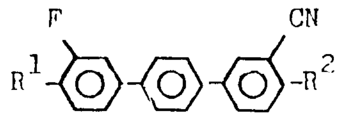

1.7

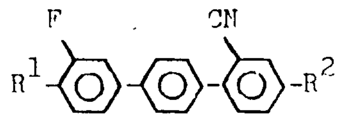

1.8

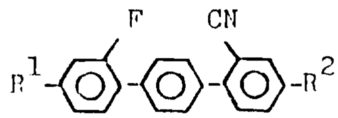

1.9

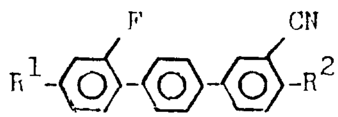

1.10

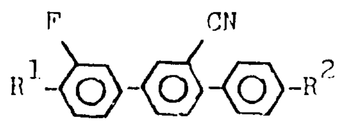

1.11

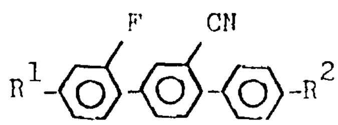

1.12

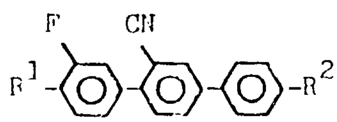

1.13

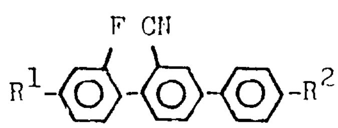

1.14

$R^1$, $R^2$ = alkyl, alkoxy or alkynyl

of the structures listed in Table 1, structures 1.1, 1.3, 1.4, 1.5, 1.6 and 1.18 are particularly preferred, especially 1.1, 1.3, 1.4, 1.10.

In compounds of formula I, IA or as listed in Table 1, $R^1$ and $R^2$ are preferably independently n-alkyl, n-alkoxy or n-alkynyl containing 3-12 especially 3-10 carbon atoms, or branched or asymmetrically substituted alkyl, alkoxy or alkynyl, which may be in an optically active or racemic form, but especially n-alkyl or n-alkoxy. $R^1$ and $R^2$ may be the same or different.

In a compound of formula 1, $R^1$ and $R^2$ may therefore be independently selected from the following preferred groups listed in Table 2 below:-

Table 2

| n-alkyl | n-alkoxy | n-alkynyl |
|---|---|---|
| $C_3H_7$ | $C_3H_7O$ | $C_2H_5C{\equiv}C$ |
| $C_4H_9$ | $C_4H_9O$ | $C_3H_7C{\equiv}C$ |
| $C_5H_{11}$ | $C_5H_{11}O$ | $C_4H_9C{\equiv}C$ |
| $C_6H_{13}$ | $C_6H_{13}O$ | $C_5H_{11}C{\equiv}C$ |
| $C_7H_{15}$ | $C_7H_{15}O$ | $C_6H_{13}C{\equiv}C$ |
| $C_8H_{17}$ | $C_8H_{17}O$ | $C_7H_{15}C{\equiv}C$ |
| $C_9H_{19}$ | $C_9H_{19}O$ | $C_8H_{17}C{\equiv}C$ |
| $C_{10}H_{21}$ | $C_{10}H_{21}$ | $C_9H_{19}C{\equiv}C$ |
| $C_{11}H_{23}$ | $C_{11}H_{12}O$ | $C_{10}H_{21}C{\equiv}C$ |
| $C_{12}H_{25}$ | $C_{12}H_{25}O$ | |

Preferred asymmetrically substituted alkyl and alkoxy groups are 2-methylbutyl, 2-methylbutyloxy, 3-methylpentyl, 3-methylpentyloxy, 4-methylhexyl, 4-methylpentyl, 1-methylheptyl and 1-methylheptyloxy.

Preferred asymmetrically substituted alkynyl groups are:-

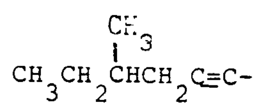

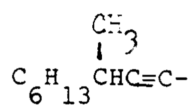

Compounds of formula I may be prepared via a number of synthetic routes, for example routes A - F shown in the accompanying figures 1-11, route B providing some useful intermediates. The respective structural types which may be prepared using these routes are as follows, referring to Table 1 above:-

| Structure | Route |
|---|---|
| 1.1 | A1, A2 |
| 1.2 | C1 |
| 1.3 | C1, C2 |
| 1.4 | D1, D2 |
| 1.5 | D3 |
| 1.6 | D4 |
| 1.7 | E1, E2 |
| 1.8 | E1 |
| 1.9 | E1 |
| 1.10 | E1, E2 |
| 1.11 | F1 |
| 1.12 | F2 |
| 1.13 | F3 |
| 1.14 | F4 |

Modifications to routes A-F to prepare compounds in which the substituents $R_1$ and $R_2$ of Table 1 are different to those illustrated in figures 1 to 11 will be apparent to those skilled in the art of organic synthesis, for example the replacement of the alkyl or alkoxy groups R, $R^1$, $R^2$ R'' by their perfluorinated analogues or by alkynyl or alkynyloxy groups.

Although the overall routes A-F and their end products are novel the individual steps use known reactions.

For example the following steps use the known coupling reaction between the halo-phenyl ring and the phenylboronic acid in the presence of tetra-(triphenylphosphine)-palladium (0): A11, A23, A24, C14, C23,

C25, D13, D22, D42, D44, E12, E22, E23, F11, F21, F31, F32, F43, F44.

The phenylboronic acids used in these steps may in turn be produced from the halophenyl precursor by the known method which first prepares a Grignard Reagent using Magnesium in tetrahydrofuron (THF) followed by a reaction with tri-isopropylborate then hydrolysis with hydrochloric acid. This procedure is used in steps B13, B22, C13, D41, D43, E11, F42.

In routes D1, D2, D3 and D4 the lateral $CO_2H$ group is introduced into the fluorinated ring using n-Butyllithium in hexane followed by reaction with carbon dioxide then hydrolysis. The $CO_2H$ group is then converted into the corresponding amide using oxalyl chloride and ammonia, followed by dehydration using thionyl chloride.

Other known reactions used in these routes are as follows:-

A21, F45 - N-bromosuccinimide/dichloromethane 0 ° C

A22, F46 - (i) $NaNO_2$/HCl (ii) KI

B11 - Friedel Crafts reaction, RCOCl/$AlCl_3$

B12, F48 - via hydrazine hydrate (F48a = direct use)

B21, C11, D21, F41 - RBr/acetone/$K_2CO_3$

C12 - $Br_2$

C21, F47 - R-C≡CH, $ZnCl_2$; $Pd(PPh_3)_4$

C22 - $H_2$, Pd/C

Apart from standard reagents the starting materials for routes A-F are known or commercially available, eg from BDH Ltd or Fluorochem. A method for the preparation of the terphenyl starting point of route D3 is found in EP-A-0132377. In routes A-F when the starting compound may be made by one or more of the other routes this is indicated, eg (C13) in route C2 shows that this starting compound may be made via step C13.

It should also be noted that the products of steps D13, D22 and D43, and the starting compound for step D31 are protected by the claims of the Applicant's EP-0132377-B and US Patent 4,594,465.

To prepare compounds in which $R^1$ and/or $R^2$ contain fluorine, the appropriate perfluoroalkyl compounds may be used as starting points, eg in steps B11 or B22 etc.

Compounds in which $R^1$ and/or $R^2$ contain a COO or OOC group, eg RCOO- where R is alkyl may for example be prepared by removing a terminal $R^1$ or $R^2$ group which is alkoxy from a terphenyl product of formula I or IA using the known method of reaction with $BBr_3$ followed by hydrolysis to leave a terminal -OH group, followed by esterification of this phenol with a carboxylic acid RCOOH or an acyl chloride RCOCl.

The C≡C group in compounds in which $R^1$ and /or $R^2$ is alkynyl is a reactive functional group and may for example be hydrated, eg using $HgSO_4$ / $H_2SO_4$ or $BH_3$ to yield terminally ketone substituted terphenyls, eg having terminal $-CO.CH_2-R$ or $-CH_2CO.-R$ groups. Such ketones may be reduced to form the corresponding alcohols, ie $-CH(.OH)CH_2-R$ or $-CH_2CH(OH)-R$, which may themselves be esterified with a carboxylic acid to form the corresponding esters.

Alternately the ketones may be converted into cyanohydrins by reaction with HCN, leaving terminal groups of structure $-C.CN.OH-CH_2-R$ and $-CH_2-C.CN.OH-R$. Such cyanohydrins may then be esterified eg with a carboxylic acid R"-COOH where R" is alkyl to yield terphenyls with one or more terminal substituent groups of structure:

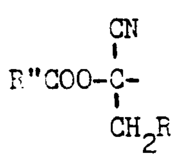

or

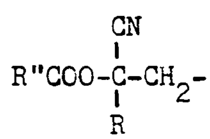

Also reaction of the C≡C group with halogens or hydrogen halides can lead to terphenyls of formula I with halogenated terminal substituents $R^1$ and / or $R^2$. For example well known reactions of an alkyne subsituent R-C≡C such as in the product of step C25 can lead in this way to $R-CX_2CX_2-$, R-CHX = CHX-, $R-CH_2CX_2-$ and $R-CX_2CH_2-$ where X is a halogen eg chlorine.

Many terphenyls of formula I show liquid crystal phases, and also a high negative dielectric anisotropy $\Delta E$. These are therefore useful components of liquid crystal materials, and therefore according to a further aspect of this invention there is provided a liquid crystal material, being a mixture of compounds, at least one being a terphenyl of formula I , preferably formula IA.

Such a liquid crystal material may be a nematic or smectic liquid crystal material.

A nematic liquid crystal material my therefore be a mixture of compounds at least one of which is a terphenyl of formula I, preferably one which exhibits a nematic liquid crystal phase. As terphenyls of

formula I have a high negative ΔE they my usefully be mixed with other compounds which show nematic liquid crystal phases and which for example have a low ΔE, or a positive ΔE to provide a mixture of intermediate ΔE. As is well known in the liquid crystal art, the sign of ΔE (ie +ve or -ve) of a nematic liquid crystal material is chosen according to the kind of electro-optical device in which the material is to be used. Hence the availability of the high negative ΔE compounds of the invention is very advantageous. In particular the terphenyls of the invention are likely to be useful in liquid crystal mixtures which use the ECB effect.

The general classes of other compounds which may be included in a nematic liquid crystal material according to this aspect of the invention will be well known to those skilled in the liquid crystal art. Some examples of compounds having a low ΔE are listed below:

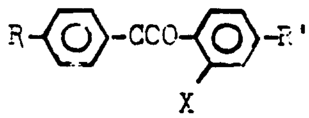
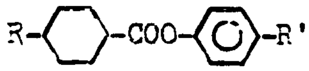
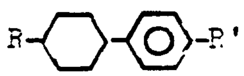
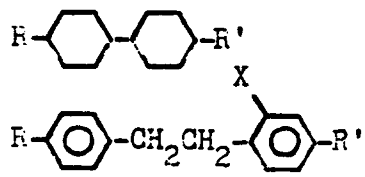
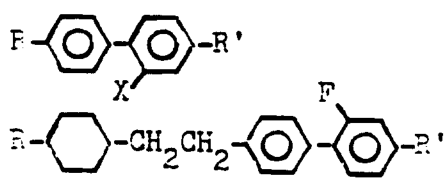

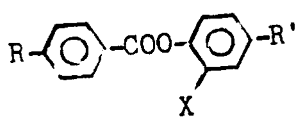
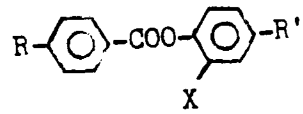
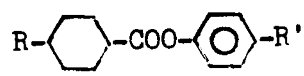
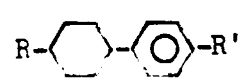
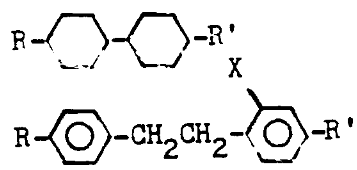
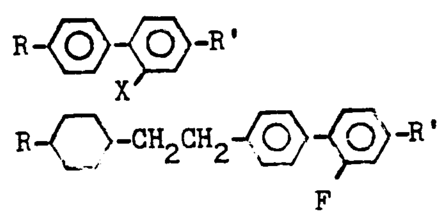

Some examples of compounds having a positive ΔE are listed below:

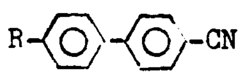
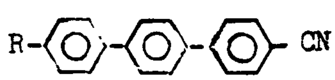
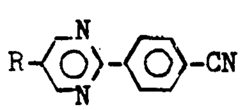
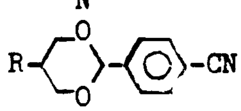
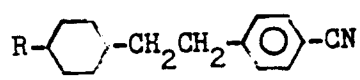
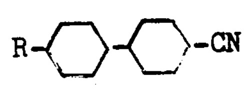
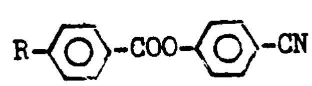

Where R and R' independently represent $C_1$ - $C_8$ alkyl or alkoxy, and X represents F or H.

The nematic liquid crystal mixture of this aspect of the invention may also contain pleochroic dyes, such as those described in EP-A-82300891, and/or one or more optically active compounds to induce the appearance of a cholesteric phase.

Typically but not exclusively a nematic liquid crystal mixture of this aspect of the invention contains:

| | |
|---|---|
| Compounds of formula I: | 5 - 95 wt % |
| Low Δ E compounds: | 0 - 95 wt % |
| High +ve Δ E compounds: | 0 - 95 wt % |
| Optically active compounds: | 0 - 5 wt % |
| Pleochroic dyes: | 0 - 5 wt % |

The overall sum of weight percentages being 100 wt %.

The invention also provides a smectic liquid crystal material which is a mixture of two or more compounds, at least one of which is a terphenyl of formula I, preferably of formula 1A, and especially of formula 1.1 or 1.4 of Table 1. This mixture preferably also contains in addition one or more other compounds which together or separately show a smectic phase. The smectic phase is preferably $S_C$, $S_F$ or $S_I$/

Preferably the mixture of this aspect of the invention contains one or more terphenyls of formula I and one or more FTP's of formula II :

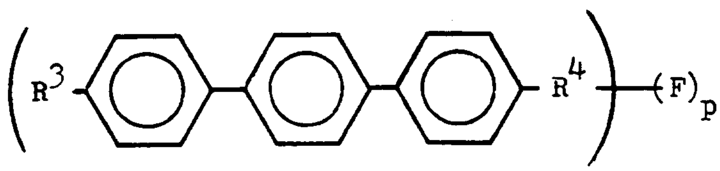

Formula II

where $R^3$ and $R^4$ are independently selected from hydrogen, alkyl and alkoxy each containing 1-12 carbon atoms, p may be 1 or 2 and the fluorosubstituents(s) may occupy any of the available lateral substitution positions. Preferred FTP's for this aspect of the invention have a formula IIA, IIB or IIC.

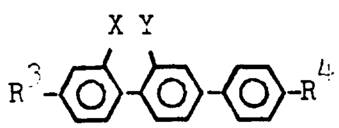

Formula IIA

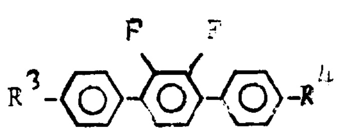

Formula IIB

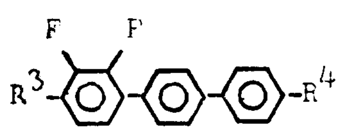

Formula IIC

where $R^3$ and $R^4$ are as defined above, and where X and Y are independently selected from hydrogen and fluorine, at least one of X and Y being fluorine. Preferably $R^3$ and $R^4$ are n-alkyl or n-alkoxy containing 3-12 carbon atoms, especially 3-10.

$R^3$ and $R^4$ may be the same or different. Preferred n-alkyl and n-alkoxy groups from which $R^3$ and $R^4$ may be selected are those from which $R^1$ and $R^2$ are selected, as listed above, although the combinations of $R^1$ and $R^2$ need not be the same as $R^3$ and $R^4$ in the mixture.

Preferably X in formula IIA is hydrogen and Y is fluorine. The preparation of such FTPs is described for example in EP-A-0132377. The preparation of FTPs in which p is 2, eg of formula IIB and IIC is described

in GB-A-8806220 filed 16-03-88, the contents of which are incorporated herein by reference.

The smectic liquid crystal material of this aspect of the invention may for example show an Sc phase at room temperature, and may be a mixture of one or more FTPs of formula IIA plus one or more terphenyls of the invention, or may be a mixture of one or more FTP's of formula II B and or one or more FTP's of formula IIC plus one or more terphenyls of the invention.

The addition of one or more compounds of formula I to one or more FTPs of formula II often results in suppression of undesirable $S_B$ phases shown by the FTP, and may also result in a mixture showing an $S_C$ phase over a broader temperature range than the FTP. This is particularly so with FTP's of formula IIA. These effects may be manifested at quite low ratios of formula I compound(s) to FTP's, for example typically in the range 1:20 to 1:5 by weight % of formula I compound(s) to FTP(s).

Other compounds which show an $S_C$ phase and which may be used in mixtures of this aspect of the invention are the known compounds:

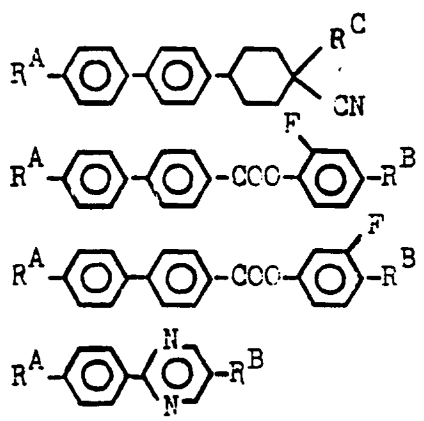

IIIA

IIIB

IIIC

IIID

where $R^A$, $R^B$ and $R^C$ independently contain 1 - 12 carbon atoms, $R^A$ and $R^B$ are independently n-alkyl or n-alkoxy, and $R^C$ is independently n-alkyl.

With the smectic mixtures of this aspect of the invention may be mixed one or more optically active compounds which induce the mixture to show an $S_C^*$ phase and/or increase the $P_S$ of the mixture. The mixtures produced in this way are ferroelectric mixtures showing a $P_S$ value and are a further aspect of this invention. The use of terphenyls of the invention in such mixtures may result in advantageous $P_S$ values and switching times.

Many optically active compounds are known which may be used in this way. Preferred optically active compounds are those described in PCT GB 85/0512, eg

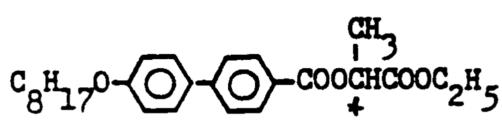

IV

the compounds described in EP-A-0110299, eg

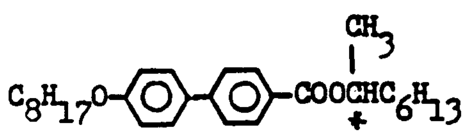

V

the compounds described in PCT/GB 87/00280, eg

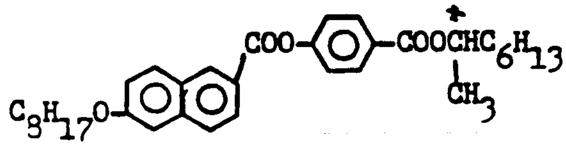

VI

and in particular the compounds described in PCT/GB87/00441 which contain a COOC*H(CN)R group where R is alkyl, for example:

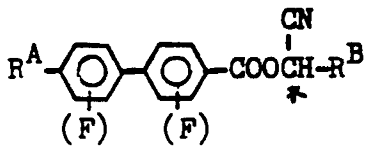

VII

where $R^A$ is n-alkyl or n-alkoxy containing 5-12 carbon atoms, (F) indicates that the phenyl ring may carry a fluoro substituent, and $R^B$ is $C_1$-4 n-alkyl, cycloalkyl or a branched alkyl of formula:

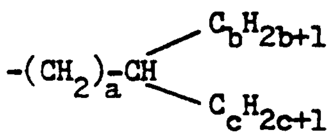

where a may be 0 or an integer 1-6, and each of b and c is an integer 1-6, preferably a being 0 and at least one of b or c being 1.

Preferred groups $R^B$ in compounds VII are methyl, $-CH(CH_3)_2$, $-CH.CH_3.CH_2CH_3$ and cyclohexyl.

Additives if used in such mixtures may fulfil a number of functions. For example additional optically active compounds may be included to control the pitch of the $S_C^*$ phase such as the optically active amide compounds described in PCT GB 87/00223, eg

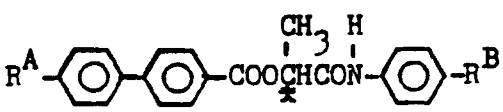

VIII

or the optically active terphenylsdescribed in GB 8703103 eg

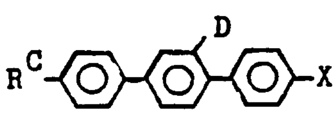

IX

where $R^A$, $R^B$ and $R^C$ are independently n-alkyl or n-alkoxy containing 1-12 carbon atoms, D is F or Cl, X is $OOCCH(CH_3)OR''$, $OCH(CH_3)COOR''$ or $COOCH(CH_3)R''$ where R'' is n-alkyl containing from 1 to 12 carbon atoms.

Additives may also serve the function of encouraging the formation of an $S_A$ phase at a temperature above that of the $S_C^*$, to assist in alignment of the mixture with the electrodes of an electro-optic device.

Additives may also suppress undesirable phases such as $S_A$ or $S_B$ so that these occur at temperatures far away from the working temperature range, eg

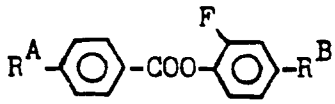

X

where $R^A$ and $R^B$ are independently n-alkyl or n-alkoxy containing 1-12 carbon atoms.

Typically but not exclusively a smectic C liquid crystal material of this aspect of the invention is a mixture having a composition as follows:

| | |
|---|---|
| One or more compounds which show together or separately a room temperature $S_C$ phase, especially of formulae IIA, IIB IIC or IIIA | 50 - 95 wt % |
| Compound(s) of formula I. | 5 - 50 wt % preferably 5 - 25 wt % |
| Optically active compound(s), at least some being present if the mixture is to show an $S_C$ phase. | 0 - 25 wt % |
| Additive(s) | 0 - 20 wt % |
| Total | 100 wt % |

The nematic and ferroelectric smectic liquid crystal mixtures of the invention may be used in any of the known types of liquid crystal electro-optical display device which use such materials, for example as described in the two Appl. Phys. Lett. references mentioned above.

The construction and method of operation of such liquid crystal electro-optical devices is well known. Generally such a device comprises two substantially parallel substrates, at least one of which is optically transparent, and having electrodes on their facing surfaces, and sandwiched between them a liquid crystal material. The application of a voltage across the liquid crystal material via the electrodes causes a change in the optical properties of the liquid crystal material to produce a visible effect. Suitable dimensions, voltages and other parameters for such a device will be apparent to those skilled in the art.

The invention will now be described by way of example only with reference to the accompanying figures 1 - 11 which show preparative routes for terphenyls of the invention and figure 12 which shows a cross section through a liquid crystal device.

In the following examples all temperatures are in °C. The abbreviations N = nematic, $S_A$ = smectic A, $S_C$ = smectic C, $S_?$ and $S_{??}$ unidentified smectic phase, I = isotropic liquid, K = solid crystal. Liquid crystal transitions shown ( ) are virtual transitions seen on supercooling.

Preparative Examples

Example 1

Using routes A1, A2, B1 and B2 the following compounds of structure 1.1 were made:

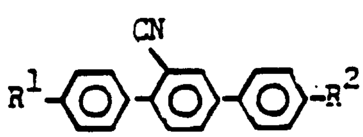

| $R^1$ | $R^2$ | Liquid crystal transition temps (°C) |
|---|---|---|
| n-$C_5H_{11}$ | n-$OC_6H_{13}$ | K 35.5 $S_A$ 97.5 I |
| n-$C_5H_{11}$ | n-$OC_8H_{17}$ | K 33.5 $S_A$ 103.0 I |
| n-$C_6H_{13}O$ | n-$C_5H_{11}$ | K 48.5 ($S_C$ 29.5) N 77.5 I |
| n-$C_8H_{17}O$ | n-$C_5H_{11}$ | K 35 0 $S_C$ 42.0 N 73.0 I |
| n-$C_6H_{13}O$ | n-$OC_6H_{13}$ | K 101.5 ($S_C$ 61.0) $S_A$ 110.0 N 114.0 I |
| n-$C_5H_{11}$ | n-$C_5H_{11}$ | $S_A$-N (33.5) N-I (38.0) K-I 40.0 |
| n-$C_6H_{13}$ | n-$OC_6H_{13}$ | |

Route B1

Step B 11

The compound

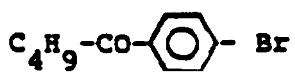

was prepared by a Friedel - Crafts reaction between bromobenzene and n-pentanoyl chloride ($C_4H_9COCl$) in the presence of aluminium chloride.

Step B 12

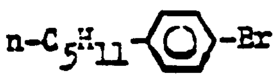

A mixture of the product of step B11 (77.1g, 0.32 mol), hydrazine hydrate (46.4g, 0.93 mol) and potassium hydroxide (59.0g, 1.05 mol) in diethylene glycol (250 ml) was heated at 130°C for 2h. The excess of hydrazine hydrate was distilled off and the temperature was raised to 200°C for 2h. The cooled mixture was poured into 18% hydrochloric acid, the product was extracted into ether (twice), and the combined ethereal extracts were washed with water and dried ($MgSO_4$). The solvent was removed in vacuo and the residue was distilled to yield a colourless liquid. The identity of the product was confirmed by nmr, ir and MS. Yield 58.1g (80%), bp 145-148°C at 2700 N $m^{-2}$ (20mm Hg).

Step B13

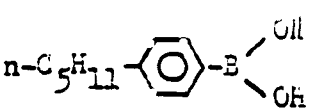

A solution of the Grignard reagent prepared from the product of step B12 (24.0g, 0.11 mol) and magnesium (2.95g, 0.12 mol) in dry THF (85 ml) was added dropwise to a stirred, cooled (-78°C) solution of tri-isopropyl borate(39.8g, 0.21 mol) in dry THF (25 ml) under dry nitrogen. The stirred mixture was allowed to warm to room temperature overnight and stirred for 1h with 10% hydrochloric acid (120 ml) at room temperature. The product was extracted into ether (twice), and the combined etheral extracts were washed with water and dried ($MgSO_4$). The solvent was removed in vacuo to afford a soft off-white solid. Yield 19.3g (95%). The identity of the product was confirmed as above.

Route B2

Step B21

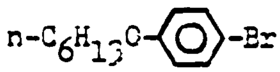

A solution of 1-bromohexane (60.0g, 0.36 mol) in acetone (150ml) was added dropwise to a stirred mixture of 4-bromophenol (71.0g, 0.41 mol) and potassium carbonate (120.0g, 0.87 mol) in acetone (600 ml) at room temperature. The stirred mixture was heated under reflux (90-95 °C) for 43 h (ie until glc analysis revealed an absence of 1-bromohexane). The product was extracted into ether (twice), and the combined ethereal extracts were washed with water, 5% sodium hydroxide, water and dried ($MgSO_4$). The solvent was removed in vacuo and the residue was distilled to yield a colourless liquid. Yield 79.4g (86%), bp 100-110 °C at 13.3 N $m^{-2}$ (0.1mm Hg). The identity of the product was confirmed as above.

Step B22

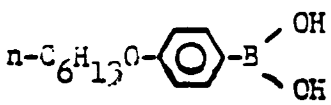

A solution of the Grignard reagent, prepared from the product of step B21 (72.0g, 0.28 mol) and magnesium (7.75g, 0.32 mol) in dry THF (220 ml) was added dropwise to a stirred, cooled (-78 °C) solution of tri-isopropyl borate(109.1g, 0.58 mol) in dry THF (40 ml) under dry nitrogen. The stirred mixture was allowed to warm to room temperature overnight and stirred for 1h with 10% hydrocholoric acid (320 ml) at room temperature. The product was extracted into ether (twice), and the combined ethereal extracts were washed with water and dried ($MgSO_4$). The solvent was removed in vacuo to afford a colourless solid. Yield 61.2g (99%) mp 80-85 °C. The identity of the product was confirmed as above.

Route A1

Step A11

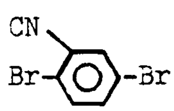

Bromine (44.0g, 0.275 mol) was added dropwise to a mixture of benzonitrile (10.0g, 0.097 mol) and aluminium chloride (42.0g, 0.31 mol) at room temperature. The mixture was heated at 70 °C for 3.5h and poured into ice/water. The product was extracted into ether (twice) and the combined ethereal extracts were washed with sodium thiosulphate and dried ($MgSO_4$). The solvent was removedin vacuo to afford an off-white solid (23.4g) (glc analysis revealed presence of three components) which was recrystallised from benzene to give colourless crystals. Yield 3.1g (12%). mp 144-145 °C (lit 144-145 °C). The identity of the product was confirmed as above.

Note: The low yield was probably due to insufficient reaction time or too low a temperature being used as glc analysis of the crude product revealed the presence of starting material and what is suspected to be 3-bromobenzonitrile.

Step A12

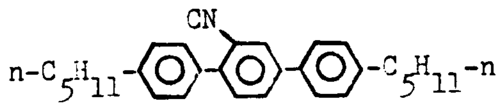

A solution of 4-pentylphenylboronic acid (B13 ) (2.88g, 0.015 mol) in ethanol (50 ml) was added dropwise to a stirred mixture of 2,5-dibromobenzonitrile (1.40g, 5.36 mol) and tetrakis-(triphenylphosphine) palladium(0) (0.3749g, 0.325 mmol) in benzene (30 ml) and 2M-sodium carbonate (30 ml) at room temperature under dry nitrogen. The stirred mixture was heated under reflux (90-95 °C) for 18h (ie until glc analysis revealed absence of starting material), cooled and stirred for 1hr at room temperature with 30% hydrogen peroxide (5 ml). The mixture was filtered, the filtrate was washed with ether (twice), and the combined ethereal phases were washed with water and dried ($MgSO_4$). The solvent was removed in vacuo and the residue was purified by column chromatography [silica gel/petroleum fraction (bp 40-60 °C) - dichloromethane, 2:1] to give a colourless solid which was recrystallised from ethanol to yield fine colourless crystals. Yield 1.13g (53%). The identity of the product was confirmed as above. The product showed the following liquid crystal transitions ( °C):
K-I 40.0, $S_A$-N (33.5), N-I (38.0)

Step A12

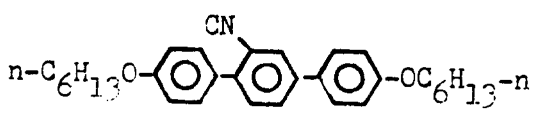

A solution of 4-hexoxyphenylboronic acid ( B 22 ) (3.33g, 0.015 mol) in ethanol (70 ml) was added dropwise to a stirred mixture of 2,5-dibromobenzonitrile (1.30g, 4.98 mol) and tetrakis-(triphenylphosphine) palladium (0) (0.3554g, 0.308 mmol) in benzene (30 ml) and 2M-sodium carbonate (30 ml) at room temperature under dry nitrogen. The stirred mixture was heated under reflux (90-95 °C) for 21h (ie until glc analysis revealed absence of starting material), cooled and stirred for 1hr at room temperature with 30% hydrogen peroxide (5 ml). The mixture was filtered, the filtrate was washed with ether (twice), and the combined ethereal phases were washed with water and dried ($MgSO_4$). The solvent was removed in vacuo and the residue was purified by column chromatography [silica gel/petroleun fraction (bp 40-60 °C)-dichloromethane, 2:1] to give a colourless solid which was recrystallised from ethanol to give colourless crystals. Yield 0.87g (38%).

Route A2

The 4-alkyl-and 4-alkoxy-phenylboronic acids prepared using route B exemplified above were used to prepare compounds of formula 1.1 via route A 2.

Step A21

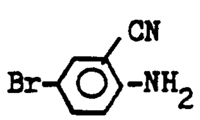

N-Bromosuccinimide (NBS) (37.71g, 0.21 mol) was added in small portions over 40 mins to a stirred, cooled (-10-0 °C) solution of 2-aminobenzonitrile (25.00g, 0.21 mol) in dry dichloromethane (150 ml) under dry nitrogen. The mixture was stirred at 0 °C for 1 h 10 mins (glc analysis revealed a complete reaction)

and washed with a large amount of water. The aqueous layer was washed with dichloromethane and the combined organic phases were washed with water and dried ($MgSO_4$). The solvent was removed in vacuo to give a red/purple solid (39g, 94%) mp = 92-94 °C.

Step A22

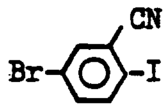

A stirred mixture of 2-amino-5-bromobenzonitrile (25.00g 0.13 mol) and 36% (conc) HCl (110 ml) was gently warmed to obtain a solution, then cooled to -5 °C and a solution of sodium nitrite (10.50g, 0.15 mol) in water (50 ml) was added dropwise whilst maintaining the tempeature at -5 °C. The mixture was stirred at 0 °C for ½ hr, cyclohexane was added, and a solution of potassium iodide (43.5g, 0.26 mol) was added dropwise at between 5 and 10 °C. The mixture was stirred at room temperature for a few hours (or overnight for convenience), then warmed gently for 10 mins to ensure complete reaction. The product was extracted into ether (twice), the combined ethereal phases were washed successively with sodium metabisulphite, 10% sodium hydroxide, water and dried ($MgSO_4$). The solvent was removed in vacuo to give a sand-coloured solid (35g, 87%). mp = 113-114 °C

Step A23

(1) 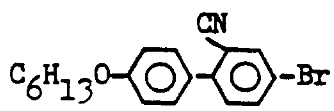

A solution of 4-hexoxyphenylboronic acid ( see step B22) ( 1.56g, 7.03 mmol) in ethanol (minimum amount possible ≏ 20 ml) was added dropwise to a stirred mixture of 5-bromo-2-iodobenzonitrile (1.80g, 5.84 mmol) and tetrakis (triphenylphosphine) palladium (0) (abbreviated hereinafter to TTP) (0.35 72g, 0.31 mmol) in benzene (30 ml) and 2M-sodium carbonate (30 ml). The stirred mixture was heated under reflux ( 95 °c) for 1¾ hr (ie until glc analysis revealed a satisfactory starting material - product ratio (1:10)). The product was extracted into ether (twice), the combined ethereal extracts were washed with brine and dried ($MgSO_4$). The solvent was removed in vacuo and the residue was purified by column chromatography (silica gel/petroleum fraction (bp 40-60 °C) - dichloromethane, 1:1) to give a colourless solid (1.72g, 82%).

(2) 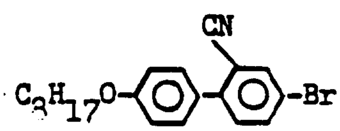

| | |
|---|---|
| 5-bromo-2-iodobenzonitrile | 2.50g, 8.12 mmol |
| 4-octoxyphenylboronic acid (via route B2) | 2.44g, 9.76 mmol |
| TTP | 0.4927g 0.43 mmol |

The method was the same as that above. The mixture was reheated under reflux (95 °C) for 2 h. Purified by column chromatography [silica gel/petroleum fraction (bp 48-60 °C) -dichloromethane, 1:1] to give a colourless solid (2.50g, 80%).

(3) 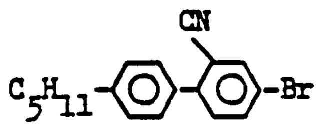

| | |
|---|---|
| 5-bromo-2-iodobenzonitrile<br>4-pentylphenylboronic acid (route B1)<br>TTP | 3.50g, 0.001 mol<br>2.53g, 0.013 mol<br>0.6421g, 0.56 mmol |

The method was the same as that above. The mixture was heated under reflux (295 ° C) for 2 h. Purified by column chromatography (silica gel/petroleum fraction (bp 40-60 ° C)-dichloromethane, 1:1) and distillatior (Kugel rohr, 13.3 M $m^{-2}$ (0.1mm Hg)) to give a pale yellow liquid (2.67 g, 74 %).

Step A24

(1) 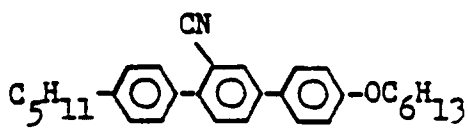

| | |
|---|---|
| 4-bromo-2-cyano-4$^1$-pentylbiphenyl (A23(3))<br>4-hexoxyphenylboronic acid (B22)<br>TTP | 0.34g, 1.0 mmol<br>0.44g, 1.98 mmol<br>0.1645g, 0.14 mmol |

(2) 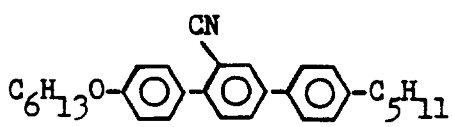

| | |
|---|---|
| 4-bromo-2-cyano-4$^1$-hexoxybiphenyl ( A23(1))<br>4-pentylphenylboronic acid (B13)<br>TTP | 1.50g, 4.19 mmol<br>1.05g, 5.47 mmol<br>0.314g, 0.27 mmol |

(3) 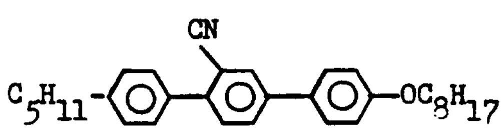

| | |
|---|---|
| 4-bromo-2-cyano-4[1]-pentylbiphenyl (A23(3)) | 1.30g, 3.96 mmol |
| 4-octoxypenylboronic acid (via route B22) | 1.19g, 4.76 mmol |
| TTP | 0.265g, 0.23 mmol |

(4) 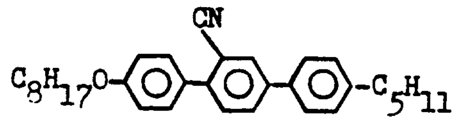

| | |
|---|---|
| 4-bromo-2-cyano-4[1]-octoxybiphenyl (from A23(2) | 1.48g, 3.83 mmol |
| 4-pentylphenylboronic acid (from B13) | 1.01g, 5.26 mmol |
| TTP | 0.2812g, 0.34 mmol |

In each case the method used was that of step A23 above. The mixture in case (2) was heated under reflux for 17 hours, in cases (1) and (3) for 21 hours, and case (4) for 22 hours, ie until glc analysis revealed a complete reaction. The product was purified by column chromatography (silica gel / petroleum fraction (bp 40 - 60) - dichloromethane 1:1) to afford solids which were recrystallised from ethanol. The product of (2) was pale yellow and so was decolourised with charcoal.

Yields:

(1) colourless crystals (0.26 g, 61 %)
(2) colourless plates (0.80 g, 44 %)
(3) colourless crystals (1.18 g, 66 %)
(4) colouless solid (1.16 g, 67 %)

Example 2

Using route C1 the following compounds of structure 1.3 were prepared:

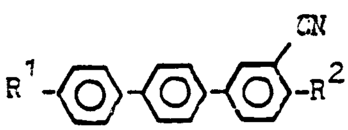

| $R_1$ | $R_2$ | Liquid Crystal Transition Temp |
|---|---|---|
| n-$C_5H_{11}$ | $OC_8H_{17}$-n | K 62.0 SA 160 I |
| n-$C_5H_{11}$ | $OC_6H_{13}$-n | K 62.0 SA 163.5 I |

Step C11

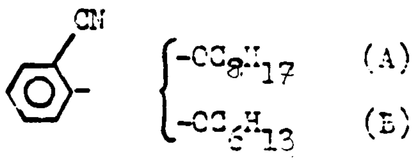

| | |
|---|---|
| 2-hydroxybenzonitrile | A 7.50g, 0.063 mol<br>B 8.10g, 0.068 mol |
| 1-bromo-octane<br>1-bromohexane | A 14.00g, 0.127 mol<br>B 13.50g, 0.082 mol |
| Potassium Carbonate | A 17.50g 0.127 mol<br>B 18.77g 0.136 mol |

The experimental procedure was the same as for Step B21 described above. Yield: A 13.90g (96%) bp 130-135° at 6.7 N $m^{-2}$ (0.05mm Hg); B 13.11g (95%) bp 115-118° at 13.3 N $m^{-2}$ (0.1mm Hg).

Step C12

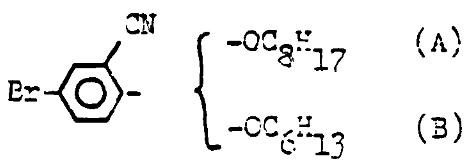

| | |
|---|---|
| Bromine | A 18.70g, 0.117 mol<br>B 19.32g 0.12 mol |
| C11 product | A 13.50g, 0.058 mol<br>B 12.25g, 0.060 mol |

The bromine was added dropwise during 15 minutes to a stirred solution of the C11 product in chloroform (30 ml) at room temperature. The stirred solution was refluxed for 42 hours (glc showed a complete reaction). The cooled solution was washed with sodium metabisulphite, water, then dried (Mg $SO_4$). The solvent was removed in vacuo in give an off-white solid. Yield: A 17.50g (97%) mp 36-37°, B 16.10g (95%) low melting around 15°.

Step C13

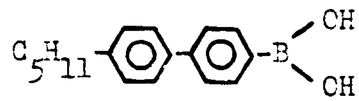

| | |
|---|---|
| 4'-bromo - 4 - pentylbiphenyl<br>magnesium<br>tri-isopropyl borate | 9.35g<br>0.871g<br>11.66g |

The experimental procedure was the same as for steps B13 and B22. The crude yield was 8.29g, 100%.

Step C14

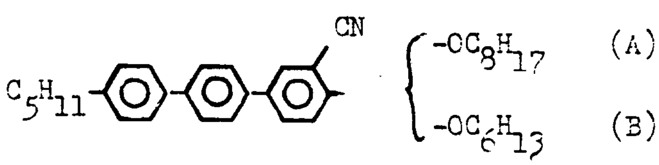

| | | |
|---|---|---|
| (A) | C12 product (A)<br>C13 product<br>TPP | 1.55g, 5.00 mmol<br>1.75g, 6.53 mmol<br>0.38g, 0.33 mmol |
| (B) | C12 product (B)<br>C13 product<br>TTP | 1.55g, 5.50 mmol<br>1.91g, 7.13 mmol<br>0.39g, 0.34 mmol |

The experimental procedure was the same as for Step A23 above. The crude product was purified by column chromatography (silica gel/petroleum fraction (bp 40-60°) dichloromethane 2:1) to give a colourless solid which was recrystallised from ethanol-ethylacetate (2:1) to yield colourless crystals. Yield: A 1.85g (82%), B 1.10g (47%).

Example 3

Using route E1 the compound of structure 1.10:

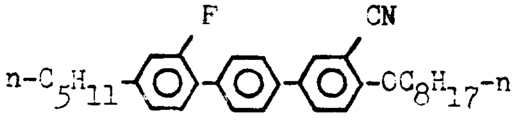

was prepared. Liquid crystal transitions were K 48.0 $S_A$ 118.0 I.

Step E11

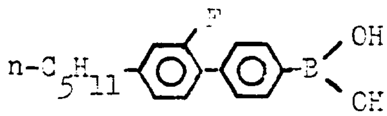

| | |
|---|---|
| 4'-bromo-2-fluro-4-pentylbiphenyl | 9.90g |
| Magnesium | 0.871g |
| Triisopropylborate | 11.66g |

The experimental procedure was the same as for Step B13 above. Crude yield: 8.85g, 100%.

Step E12

| | |
|---|---|
| 5-bromo-2-octoxybenzonitrile (C12A) | 1.30g, 4-19 mmol |
| E11 product | 1.56g, 5.45 mmol |
| TTP | 0.28g, 0.24 mol |

The experimental procedure was the same as for step A23 above. The crude product was purified by column chromatography (silica gel/petroleum fraction (bp 40-60°) dichloromethane 2:1) to give a colourless solid which was recrystallised from ethanol to give colourless crystals.

Example 4

Using route D2 the following compounds of structure 1.4 was prepared:

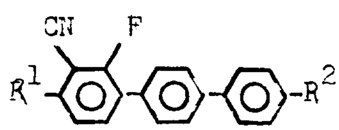

| $R_1$ | $R_2$ | Liquid Crystal Transition Temps |
|---|---|---|
| n-$C_6H_{13}$O- | n$C_5H_{11}$ | K 100.5 $S_A$ 167.0 I |
| n-$C_8H_{17}$O- | n$C_5H_{11}$ | K 100.0 $S_A$ 163.5 I* |

* The extrapolated dielectric anistropy ΔE of the compound was measured to be - 9.5.

Step D21

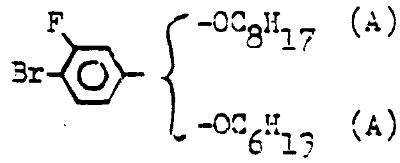

| | | |
|---|---|---|
| (A) | 4-bromo-3-fluorophenol<br>1-bromo-octane<br>potassium carbonate | 6.00g<br>7.30g<br>10.00g |
| (B) | 4-bromo-3-fluorophenol<br>1-bromohexane<br>potassium carbonate | 10.00g<br>10.37g<br>14.50g |

The experimental procedure was the same as for Step B21 above. Yields: A 9.01g, 96% as a colourless liquid bp 140-142° at 66.7 N $m^{-2}$ (0.5mm Hg); B 13.71g, 96%.

Step D22

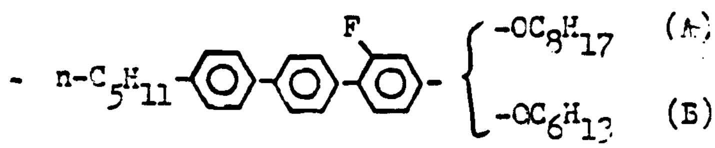

| | | |
|---|---|---|
| (A) | Step D21 product<br>Step C13 product<br>TTP | 2.00g, 6.60 mmol<br>2.30g, 8.58 mmol<br>0.60g, 0.52 mmol |
| (B) | Step D21 product<br>Step C13 product<br>TIP | 1.50g, 5.45 mmol<br>1.90g, 7.10 mmol<br>0.50g 0.43 mmol |

The experimental procedure was the same as for Step A23 above. The crude products were purified by column chromatography (silica gel/petroleum fraction (bp 40-60°) dichloromethane, 3:1) to yield colourless solids which were recrystallised from ethanol-ethyl acetate (2:1) to yield colourless crystals. Yield: A 2.30g, 78%; B 1.46g, 64%.

These products showed liquid crystal phases as below:

(A) K 69.0 ($S_K$ 25.0 $S_J$ 43.0) $S_C$ 119.0 N 158.0 I

(B) K 83.5 ($S_K$ 48.5 $S_J$ 62.0) $S_C$ 105.0 N 166.0 I

Step D23

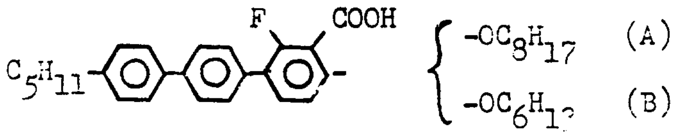

| | | |
|---|---|---|
| (A) | Step D22 product<br>n-butyl lithium | 1.50g, 3.36 mmol<br>0.50 ml 10.0M in hexane, 5.00 mmol |
| (B) | Step D22 product<br>n-butyl lithium | 1.25g, 2/99 mmol<br>1.20 ml 2.5M in hexane, 3.00 mmol |

The soloution of n-butyl lithium was added dropwise to the solution of the appropriate D22 product in dry THF (80 ml) under dry nitrogen with stirring, cooled to -78 °C. In the case of (A) the mixture was maintained under these conditions for 6 hours, and for (B) for 5 hours. The mixture was then poured into a solid $CO_2$/ether slurry. 10% hydrochloric acid was added, the aqueous layer was washed with ether and the combined ethereal extracts were washed with water and dried (Mg $SO_4$). The solvent was removed in vacuo to give colourless solids. Yields: (A) 1.65g, (B) 1.45g (tlc analysis revealed the presence of some starting materials).

Step D24

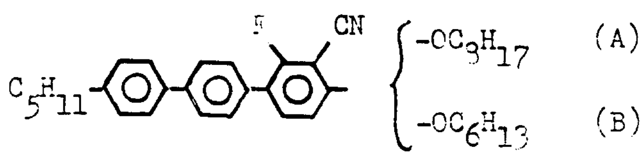

| | | |
|---|---|---|
| (A) | Step D23 product<br>oxalyl chloride<br>35% ammonia<br>DMF<br>thionyl chloride | 1.65g 3.37 mmol<br>1.00g 7.87 mmol<br>30 ml<br>8 drops<br>4.10g, 0.034 mol |
| (B) | Step D23 product<br>oxalyl chloride<br>35% ammonia<br>DMF<br>thionyl chloride | 1.45g 3.14 mmol<br>0.80g 6.30 mmol<br>25 ml<br>2 drops<br>3.70g, 0.03 mol |

A solution of the oxalyl chloride in dry benzene (30 ml) was added dropwise to a stirred solution of the Step D23 product and DMF in dry benzene (30 ml) at room temperature. The mixture was stirred at room temperature overnight and the excess of oxalyl chloride and benzene were removed in vacuo. The residue was dissolved in diglyme (10 ml) and added dropwise to the gently stirred 35% ammonia. the resulting precipitate was filtered off and dried (Ca $Cl_2$) in vacuo 13.3 $Nm^{-2}$(0.1mm Hg) to give a colourless solid. A solution of the thionyl chloride in dry DMF (30 ml) was added dropwise to a stirred solution of this solid in dry DMF (30 ml). The mixture was stirred at room temperature overnight and poured onto ice/water. The product warn extracted into ether (twice), the coined ethereal extracts were washed with water, sodium hydrogen carbonate, water and dried ($MgSO_4$). The solvent was removed in vacuo and the residue was purified by column chromatography [silica gel/petroleum fraction (bp 40-60 °C) - dichloromethane, 2:1] to

give a colourless solid. In the case of (A) this was recrystallised from ethyl acetate, and (B) from ethanol-ethyl acetate (2:1) to yield colourless crystals. Yield A: 0.21g, 13% based on D22 product; B 0.20g, 15% based on D22 product.

Examples of Liquid Crystal Mixtures

Example 5

Properties of mixtures of two cyano-terphenyls of formula 1.1, ie (A) where both $R^1$ and $R^2$ are n-pentyl, and (B) where both $R^1$ and $R^2$ are n-hexyloxy, with an FTP of formula:

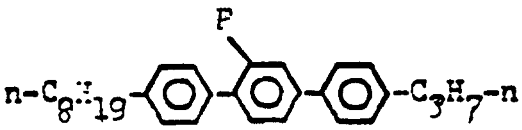

are listed below together with properties of the pure FTP.

Example 5 (contd.)

| | Transitions (°C) | | | | | | | | | | |
|---|---|---|---|---|---|---|---|---|---|---|---|
| | K | | $S_B$ | | $S_C$ | | $S_A$ | | N | | I |
| 100% FTP | • | 46 | • | (35) | • | 52.2 | • | 89 | • | 126.5 | • |
| 95% FTP )<br>5% (A) ) | • | 42.5 | • | – | • | 48 | • | 80 | • | 122 | • |
| 85% FTP )<br>15% (A) ) | • | 36 | • | – | • | 39 | • | 60.5 | • | 113 | • |
| 94% FTP )<br>6% (B) ) | • | 44 | • | – | • | 55.5 | • | 82 | • | 125 | • |
| 85% FTP )<br>15% (B) ) | • | 44 | • | – | • | 56 | • | 78 | • | 123 | • |

From these results it is clear that the mixing of a cyanoterphenyl of the invention with an FTP suppresses the $S_B$ phase of the FTP, in some cases lowers the temperature at which the $S_C$ phase of the FTP appears on heating, and in others broadens the temperature range over which the $S_C$ phase persists.

In the following examples the following mixtures and compounds are referred to by the abbreviations indicated:

Mixture H1

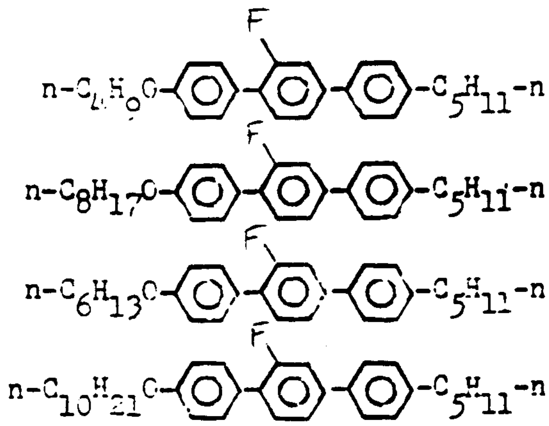

32.5 wt %

29.5 wt %

22.0 wt %

16.0 wt %

(ie a mixture of FTP's of formula IIA (Y = F) described above)

This mixtures shows liquid crystal transition temperatures:
K 31.8 $S_?$ 40.4 $S_{??}$ 50.6 $S_C$ 107 N 160 I

Mixture H2

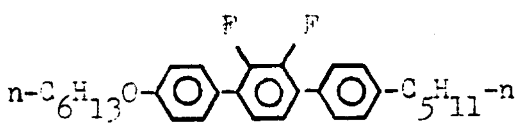

37.25 wt %

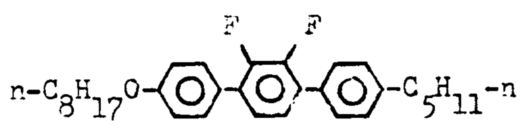

40.8 wt %

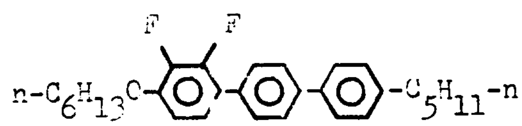

13.6 wt %

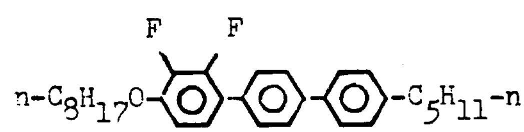

8.35 wt %

(ie a mixture of FTP's of formula IIB and IIC described above)

This mixture shows liquid crystal transition temperatures:
< 20 Sc 96.9 N 146.2 I

Mixture H3

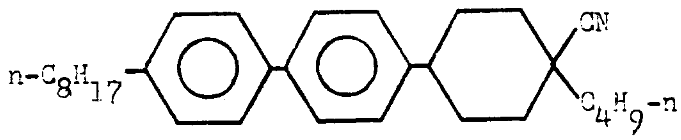

50 wt %

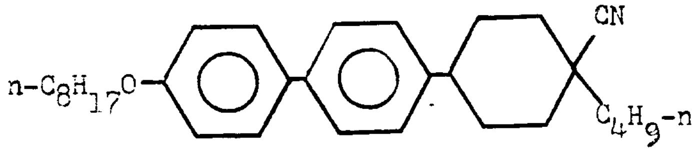

50 wt %

(ie a mixture of compounds of formula IIIA)

This mixture shows liquid crystal transition temperatures:
$S_?$ 41.5 Sc 87 $S_A$ 120.7 N 134.5 I

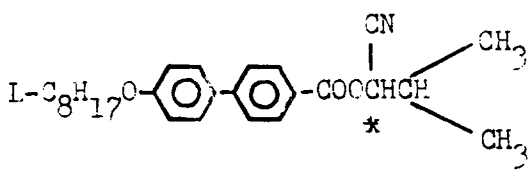

Dopant 1.

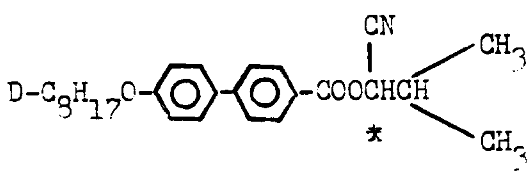

Dopant 2.

(ie formula VII described above)

Example 6

**Mixture H1 + 10%**

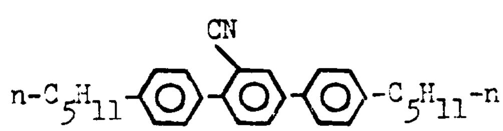

**K 13 Sc 88.4 N 134.3 I**

Addition of the cyanoterphenyl results in increased Sc range and elimination of low temperature undesirable smectic phases relative to pure H1.

Example 7

Mixture H1 + 10% 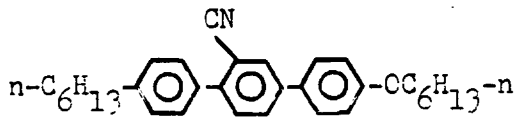

K 16.5 Sc 96.4 N 153 I

Increased Sc range, elimination of lower smectic phases.

Example 8

Mixture H3 + 10% 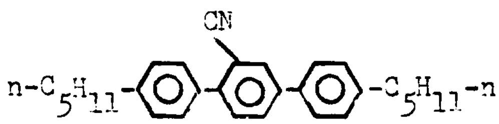

< -20 Sc 66 SA 106.6 N 124 I

Supercooled to much lower temperature than pure H3, no lower smectic phases.

Example 9

| | |
|---|---|
| Mixture H2 | 72 wt% |
| 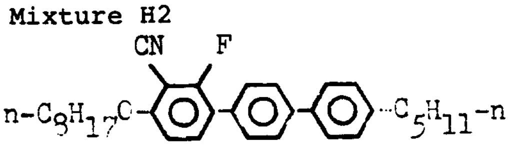 | 18 wt % |
| Dopant 1 | 6.25 wt % |
| Dopant 2 | 3.75 wt % |

< 20 $S_C^*$ 90 $S_A$ 124 N 133 I

This mixture shows a room temperature $S_c^*$ phase, with a higher temeprature $S_A$ phase which assists alignment in a liquid crystal device. Switching response time (μsec) at various peak voltages with zero volts AC bias at 30 °C, and spontaneous polarisation Ps at various temperatures using a 2 μm polyimide aligned cell are tabulated below:

| Peak voltage (v) | Resp. time | Temp (°C) | P (nC/cm$^2$) |
|---|---|---|---|
| 10 | 205 | 80 | 2.0 |
| 15 | 99 | 70 | 4.5 |
| 20 | 63 | 60 | 6.7 |
| 25 | 46 | 50 | 8.1 |
| 30 | 38 | 40 | 9.3 |
| 35 | 31 | 40 | 9.3 |
| 40 | 27 | 30 | 10.5 |
| 45 | 23 | | |
| 50 | 21 | | |
| 55 | 19 | | |
| 60 | 17 | | |
| 65 | 16 | | |

At 30°C the cone angle was found to be 23°.

Example 10

Mixture H2 72 wt %

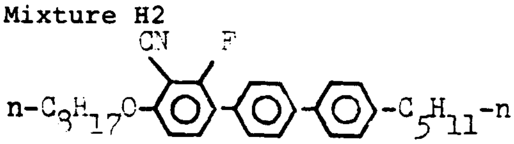

18 wt %

Dopant 1 5.8 wt %

Dopant 2 4.2 wt %

20 Sc* 91.5 SA 123.7 N 133.7 I

Switching response times (µsec) at various AC bias voltages, at 30°C using a 1.9 µm polyimide aligned cell are tabulated below. The cone angle was 23°

| Peak Voltage (v) | Resp time OV AC | Resp time 5V AC | Resp time 10V AC |
|---|---|---|---|
| 10 | 1300 | 995 | 530 |
| 15 | 287 | 283 | 280 |
| 20 | 155 | 174 | 219 |
| 25 | 127 | 130 | 192 |
| 30 | 88 | 114 | 213 |
| 35 | 77 | 125 | |
| 40 | 81 | 233 | |
| 45 | 124 | 366 | |
| 50 | 300 | 467 | |

Using a 6 µm polyimide aligned cell the Ps at various temperatures was measured and tabulated below.

| Temp (°C) | Ps (nC/cm$^2$) |
|---|---|
| 80 | 1.3 |
| 70 | 2.5 |
| 60 | 3.4 |
| 50 | 4.0 |
| 40 | 4.4 |
| 30 | 4.9 |

<u>Example 11</u> (comparative)

To illustrate the advantages of use of the terphenyls of the invention in liquid crystal materials two mixtures were prepared containing mixture H2 without a terphenyl of the invention, and proportions of dopants 1 and 2 the same as or very close to those used in the mixtures of examples 9 and 10, identified as mixtures 9A and 10A below.

| | 9A | 10A |
|---|---|---|
| Mixture H2 | 90 wt % | 90 wt % |
| Dopant 1 | 6.25 wt % | 5.63 wt % |
| Dopant 2 | 3.75 wt % | 4.37 wt % |

Their properties were:

| | | | |
|---|---|---|---|
| <u>9A</u>: | 20 $S_c^*$ 90.4 | SA 109.6 | N 133 I |
| <u>10A</u>: | 20 $S_c^*$ 93 | SA 109.6 | N 133 I |

Hence these two mixtures retain their useful $S_c^*$ phase up to virtually the same temperature as the mixtures of Examples 9 and 10 respectively.

At 30°C mixtures 9A and 10A showed the following Ps and minimum switching response times (T min) (μ sec) at the voltage (V min) and AC bias shown, the corresponding values for mixtures 9 and 10 being given for comparison.

| Mixture | OV AC | | 5V AC | | 10V AC | | Ps |
|---|---|---|---|---|---|---|---|
| | T min | V min | T min | V min | T min | V min | |
| 9 | (17 μsec at 60V, no V min observed) | | | | | | 10.5 |
| 9A | 23 | 50 | 36 | 40 | - | - | 8.96 |
| 10 | 77 | 35 | 114 | 30 | 192 | 25 | 4.9 |
| 10A | 86 | 35 | 149 | 30 | 243 | 20 | 4.24 |

From this table it can be seen that mixtures 9 and 10, which contain a terphenyl of the invention, have a higher Ps and faster switching times than the corresponding mixtures 9A and 10A which lack such a terphenyl.

An example of the use of a compound of Formula I in a liquid crystal material and device embodying the present invention will now be described with reference to the accompanying drawing, Figure 12 which is a cross sectional end view of a liquid crystal shutter.

In Figure 12a liquid crystal cell comprises a layer 1 of liquid crystal material exhibiting a nematic or chiral smectic C phase, between a glass slide 2 having a transparent conducting layer 3 on its surface, eg of tin oxide or indium oxide, and a glass slide 4 having a transparent conducting layer 5 on its surface. The slides 2,4 bearing the layers 3, 5 are respectively coated by films 6, 7 of a polyimide polymer. Prior to construction of the cell the films 6 and 7 are rubbed with a soft tissue in a given direction the rubbing directions being arranged parallel upon construction of the cell. A spacer 8 eg of polymethylmethacrylate,

separates the slides 2, 4 to the required distance, eg 5 microns. The liquid crystal material 1 is introduced between the slices 2,4 by filling the space between the slides 2, 4 and spacer 8 and sealing the spacer 8 in a vacuum in a known way.

Suitable materials for the layer 1 of liquid crystal material are the mixtures of examples 9 and 10 above, with a spacing between the films 6 and 7 of about 2 $\mu$m.

## Claims

**1.** A laterally cyano-substituted terphenyl characterised by a general formula:

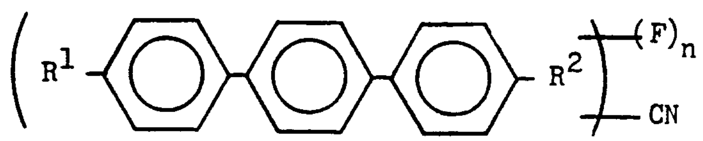

I

wherein $R^1$ and $R^2$ are independently selected from hydrogen or $C_{1-15}$ alkyl, alkoxy, or alkyl or alkoxy in which one or more $CH_2$ groups are replaced by O, COO, OOC, CHX, $CX_2$, CH=CX, CX=CH, CX=CX, where X is fluorine or chlorine, CRCN where R is alkyl, or C≡C, or in which a terminal $CH_3$ of the said alkyl or alkoxy chain is replaced by $CF_3$, n is 0 or 1, and the CN and F (if present) substituent are independently located in any of the available substitution positions.

**2.** A terphenyl according to claim 1 characterised by a general formula:

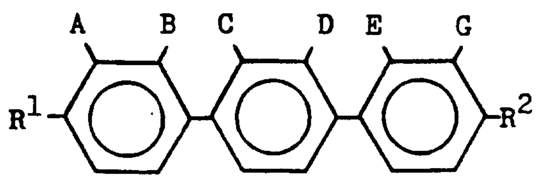

IA

wherein $R^1$ and $R^2$ are independently alkyl, alkoxy or alkynyl, and the terphenyl has a substitution pattern selected from any one of the following substitution patterns: (D = CN), (A = CN), (B = F, G = CN), (A = CN, B = F), (B = CN), (C = CN, D = F), (A = F, G = CN), (A = F, E = CN), (B = F, E = CN), (B = F, D = CN), (A = F, D = CN), (A = F, C = CN), ( B = F, C = CN), ( A = F, B = CN ) the remaining lateral substitution positions being occupied by hydrogen.

**3.** A terphenyl according to claim 2 characterised in that $R^1$ and $R^2$ are independently selected from n-alkyl or n-alkoxy containing 3 - 12 carbon atoms.

**4.** A terphenyl according to claim 3 characterised by a formula:

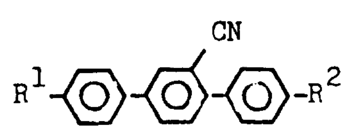

**5.** A terphenyl according to claim 3 characterised by a formula:

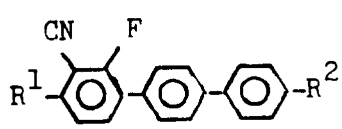

6. A terphenyl according to claim 3 characterised by a formula selected from the two below:

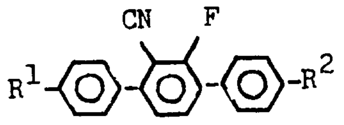
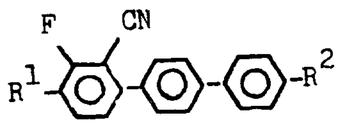

7. A terphenyl according to claim 3 characterised by a formula:

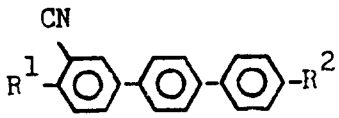

8. A terphenyl according to claim 3 characterised by a formula:

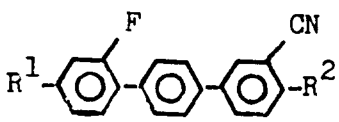

9. A liquid crystal material being a mixture of compounds, characterised in that at least one of said compounds is a terphenyl as claimed in claim 1.

10. A liquid crystal material being a mixture of compounds characterised in that at least one of said compounds is a terphenyl as claimed in claim 2.

11. A liquid crystal material according to claim 10, characterised in that at lest one of said compounds is a terphenyl of formula:

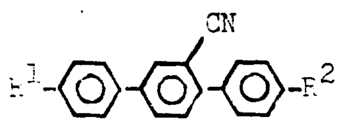

wherein $R^1$ and $R^2$ are independently $C_3$ - $C_{12}$ n-alkyl or n-alkoxy.

12. A liquid crystal material according to claim 10, characterised in that at least one of said compounds is a terphenyl of formula:

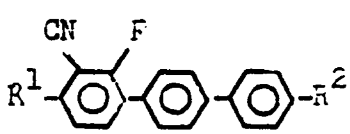

wherein $R^1$ and $R^2$ are independently $C_3$ - $C_{12}$ n-alkyl or n-alkoxy.

13. A liquid crystal material according to claim 10, characterised in that at least one of said compounds is a terphenyl having a formula selected from the two below:

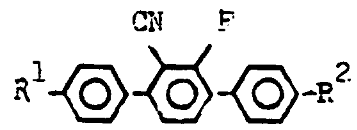

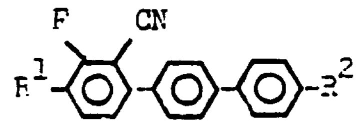

wherein $R^1$ and $R^2$ are independently $C_3$ - $C_{12}$ n-alkyl or n-alkoxy.

**14.** A liquid crystal material according to any one of claims 9 to 13 characterised in that the material additionally contains one or more fluorinated terphenyls of general formula:

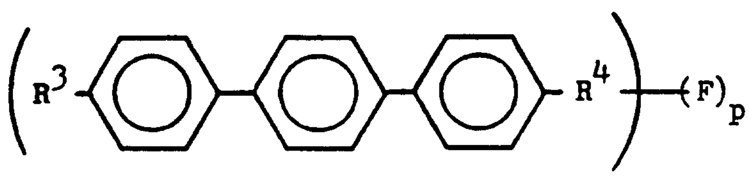

where $R^3$ and $R^4$ are independently selected from hydrogen, alkyl and alkoxy each containing 1-12 carbon atoms, p may be 1 or 2 and the fluorosubstituents(s) may occupy any of the available lateral substitution positions.

**15.** A liquid crystal material according to claim 14 characterised in that the fluorinated terphenyl has a formula selected from one or more of the following:

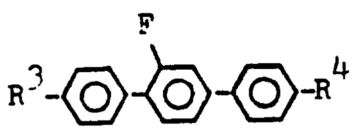

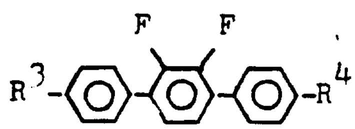

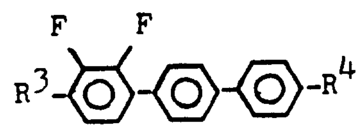

**16.** A liquid crystal material according to any one of claims 9 to 13 characterised in that the mixture additionally contains one or more compounds of formula:

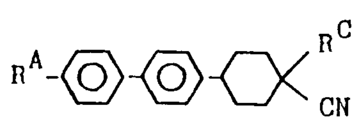

wherein $R^A$ and $R^C$ independently contain 1 - 12 carbon atoms. $R^A$ is n-alkyl or n-alkoxy, $R^C$ is n-alkyl.

**17.** A liquid crystal material according to claim 9 or claim 10 characterised in that the mixture additionally contains one or more optically active compounds of formula:

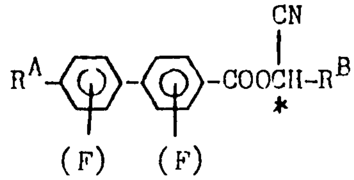

wherein $R^A$ is $C_5$ - $C_{12}$ n-alkyl or n-alkoxy; (F) indicates that the phenyl ring may carry a fluoro substituent, and $R^B$ is $C_1$ - $C_4$ n-alkyl, cycloalkyl, or a branched alkyl group of formula:

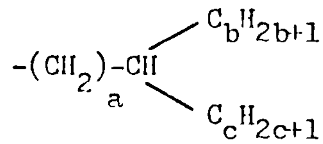

where a is 0 or an integer 1 - 6 and each of b and c is an integer 1 - 6.

**18.** Use in a liquid crystal electro optical display device in the form of two substantially parallel substrates at least one of which is optically transparent and having electrodes on their facing surfaces of a liquid crystal material as claimed in claim 9 or 10.

## Patentansprüche

**1.** Seitlich cyano-substituiertes Terphenyl, gekennzeichnet durch die allgemeine Formel:

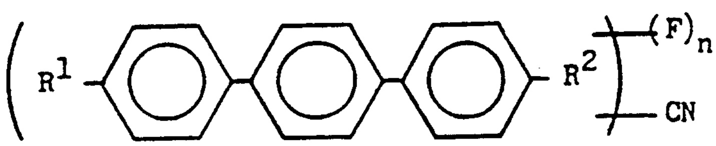

I

worin $R^1$ und $R^2$ unabhängig ausgewählt sind aus Wasserstoff oder $C_{1-15}$ Alkyl, Alkoxy oder Alkyl oder Alkoxy, in dem eine oder mehrere $CH_2$ - Gruppen ersetzt sind durch O, COO, OOC, CHX, $CX_2$, CH = CX, CX = CH, CX = CX, wobei X Fluor oder Chlor ist, CRCN, wobei R Alkyl ist, oder C≡C, oder in dem eine Endgruppe $CH_3$ der Alkyl- oder Alkoxykette durch $CF_3$ ersetzt ist, n 0 oder 1 ist und die CN- und (falls vorhanden) F-Substituenten unabhängig in beliebigen der verfügbaren Substitutionspositionen angeordnet sind.

**2.** Terphenyl nach Anspruch 1, gekennzeichnet durch die allgemeine Formel:

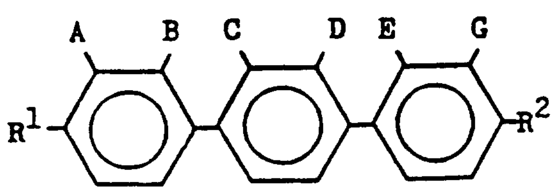

IA

worin $R^1$ und $R^2$ unabhängig Alkyl, Alkoxy oder Alkynyl sind und das Terphenyl ein Substitutionsmuster hat, das ausgewählt ist aus irgendeinem der folgenden Substitutionsmuster: (D = CN), (A = CN), (B = F, G = CN), (A = CN, B = F), (B = CN), (C = CN, D = F), (A = F, G = CN), (A = F, E = CN), (B = F, E = CN), (B = F, D = CN), (A = F, D = CN), (A = F, C = CN), (B = F, C = CN), (A = F, B = CN), wobei die verbleibenden seitlichen Substitutionspositionen von Wasserstoff eingenommen werden.

**3.** Terphenyl nach Anspruch 2, dadurch gekennzeichnet, daß $R^1$ und $R^2$ unabhängig ausgewählt sind aus n-Alkyl oder n-Alkoxy mit 3-12 Kohlenstoffatomen.

**4.** Terphenyl nach Anspruch 3, gekennzeichnet durch die Formel:

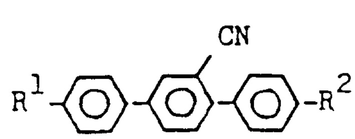

**5.** Terphenyl nach Anspruch 3, gekennzeichnet durch die Formel:

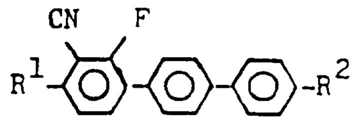

**6.** Terphenyl nach Anspruch 3, gekennzeichnet durch eine der beiden folgenden Formeln:

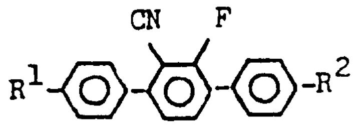

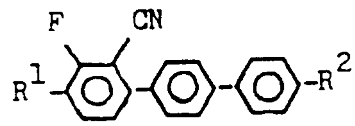

**7.** Terphenyl nach Anspruch 3, gekennzeichnet durch die Formel:

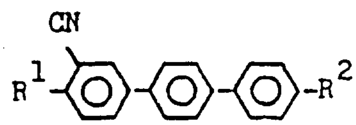

**8.** Terphenyl nach Anspruch 3, gekennzeichnet durch die Formel:

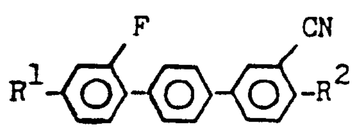

**9.** Flüssigkristallmaterial in Form eines Gemischs von Verbindungen, dadurch gekennzeichnet, daß wenigstens eine der Verbindungen ein Terphenyl nach Anspruch 1 ist.

**10.** Flüssigkristallmaterial in Form eines Gemischs von Verbindungen, dadurch gekennzeichnet, daß wenigstens eine der Verbindungen ein Terphenyl nach Anxpruch 2 ist.

**11.** Flüssigkristallmaterial nach Anxpruch 10, dadurch gekennzeichnet, daß wenigstens eine der Verbindungen ein Terphenyl der folgenden Formel ist:

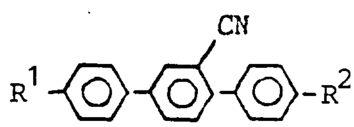

worin $R^1$ und $R^2$ unabhäbgig $C_3$ - $C_{12}$ n-Alkyl oder n-Alkoxy sind.

**12.** Flüssigkristallmaterial nach Anspruch 10, dadurch gekennzeichnet, daß wenigstens eine der Verbindungen ein Terphenyl der folgenden Formel ist:

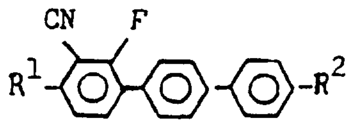

worin $R^1$ und $R^2$ unabhängig $C_3$ - $C_{12}$ n-Alkyl oder n-Alkoxy sind.

**13.** Flüssigkristallmaterial nach Anspruch 10, dadurch gekennzeichnet, daß wenigstens eine der Verbindungen ein Terphenyl gemäß einer der beiden folgenden Formeln ist:

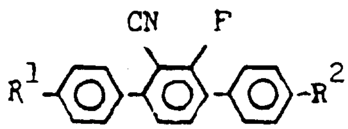

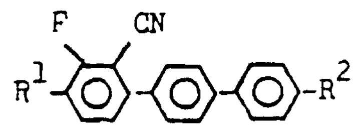

worin $R^1$ und $R^2$ unabhängig $C_3$ - $C_{12}$ n-Alkyl oder n-Alkoxy sind.

**14.** Flüssigkristallmaterial nach einem der Ansprüche 9 bis 13, dadurch gekennzeichnet, daß das Material zusätzlich ein oder mehrere fluorierte Terphenyle der folgenden allgemeinen Formel enthält:

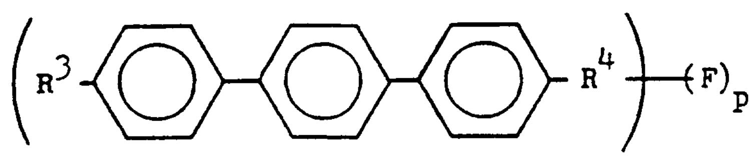

worin $R^3$ und $R^4$ unabhängig ausgewählt sind aus Wasserstoff, Alkyl und Alkoxy, von denen jedes 1-12 Kohlenstoffatome enthält, p 1 oder 2 sein kann und der oder die Fluorsubstiuenten irgendeine der verfügbaren seitlichen Substitutionspositionen einnehmen können.

**15.** Flüssigkristallmaterial nach Anspruch 14, dadurch gekennzeichnet, daß das fluorierte Terphenyl eine Formel gemäß einer oder mehrerer der folgenden Formeln hat:

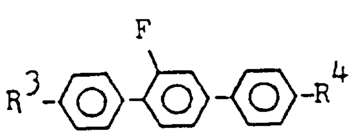

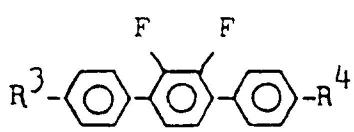

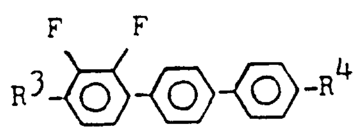

**16.** Flüssigkristallmaterial nach einem der Ansprüche 9 bis 13, dadurch gekennzeichnet, daß das Gemisch zusätzlich eine oder mehrere Verbindungen der folgenden Formel enthält:

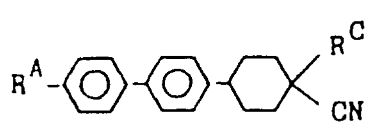

worin $R^A$ und $R^C$ unabhängig 1-12 Kohlenstoffatome enthalten, $R^A$ n-Alkyl oder n-Alkoxy ist und $R^C$ n-Alkyl ist.

**17.** Flüssigkristallmaterial nach Anspruch 9 oder 10, dadurch gekennzeichnet, das Gemisch zusätzlich eine oder mehrere optisch aktive Verbindungen der folgenden Formel enthält:

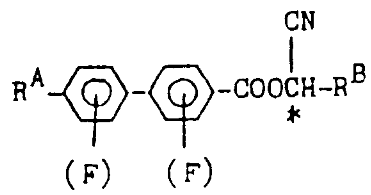

worin $R^A$ $C_5$ - $C_{12}$ n-Alkyl oder n-Alkoxy ist, (F) angibt, daß der Phenylring einen Fluorsubstituenten tragen kann, und $R^B$ $C_1$ - $C_4$ n-Alkyl, Cycloalkyl oder eine verzweigte Alkylgruppe der folgenden Formel ist:

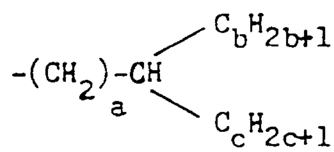

worin a 0 oder eine ganze Zahl von 1 bis 6 ist und b sowie c eine ganze Zahl von 1 bis 6 sind.

**18.** Verwendung eines Flüssigkristallmaterials nach Anspruch 9 oder 10 in einer elektrooptischen Flüssigkristallanzeigevorrichtung in Form von zwei im wesentlichen parallelen Substraten, von denen wenigstens eines optisch durchsichtig ist und deren einander zugewandte Oberflächen Elektroden aufweisen.

**Revendications**

**1.** Terphényle à substitution cyano latérale, caractérisé par une formule générale

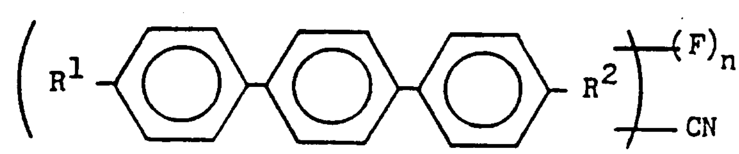

I

dans laquelle $R^1$ et $R^2$ sont choisis chacun, indépendamment, parmi un atome d'hydrogène ou un groupe alkyle, alcoxy en $C_1$ à $C_{15}$ ou bien alkyle ou alcoxy dans lequel un ou plusieurs groupes $CH_2$ sont remplacés par O, COO, OOC, CHX, $CX_2$, CH=CX, CX=CH, CX=CX, groupes dans lesquels X représente un atome de fluor ou de chlore, CRCN dans lequel R représente un groupe alkyle, ou bien C≡C, ou bien un groupe terminal $CH_3$ de ladite chaîne alkyle ou alcoxy est remplacé par $CF_3$ ; n vaut 0 ou 1, et le substituant CN et le substituant F (s'ils est présent) est ou sont, indépendamment, situés sur l'une quelconque des positions disponibles pour une substitution.

**2.** Terphényle selon la revendication 1, caractérisé par une formule générale

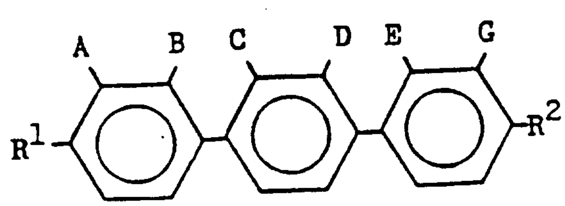

IA

dans laquelle $R^1$ et $R^2$ représentent chacun, indépendamment, un groupe alkyle, alcoxy ou alcynyle, et le terphényle a un schéma de substitution choisi parmi l'un quelconque des schémas suivants de substitution : (D = CN), (A = CN), (B = F, G = CN), (A = CN, B = F), (B = CN), (C = CN, D = F), (A = F, G = CN), (A = F, E = CN), (B = F, E = CN), (B = F, D = CN), (A = F, D = CN), (A = F, C = CN), (B = F, C = CN), (A = F, B = CN), les autres positions latérales de substitution étant occupées par des atomes d'hydrogène.

3. Terphényle selon la revendication 2, caractérisé en ce que $R^1$ et $R^2$ sont choisis chacun, indépendamment, parmi un groupe n-alkyle ou n-alcoxy contenant 3 à 12 atomes de carbone.

4. Terphényle selon la revendication 3, caractérisé par une formule :

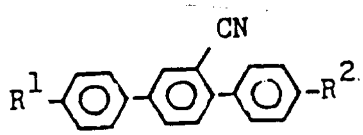

5. Terphényle selon la revendication 3, caractérisé par une formule

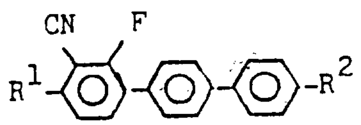

6. Terphényle selon la revendication 3, caractérisé par une formule choisie parmi les deux ci-après

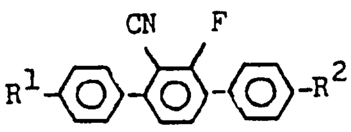

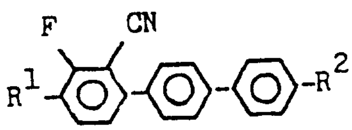

7. Terphényle selon la revendication 3, caractérisé par une formule

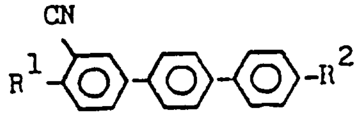

8. Terphényle selon la revendication 3, caractérisé par une formule

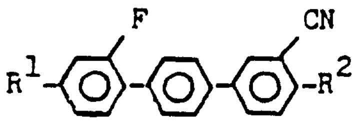

**9.** Matière à propriétés de cristaux liquides, qui est un mélange de composés, matière caractérisée en ce qu'au moins l'un desdits composés est un terphényle tel que revendiqué à la revendication 1.

**10.** Matière à propriétés de cristaux liquides, qui est un mélange de composés, matière caractérisée en ce qu'au moins l'un desdits composés est un terphényle tel que revendiqué à la revendication 2.

**11.** Matière à propriétés de cristaux liquides selon la revendication 10, caractérisée en ce qu'au moins l'un desdits composés est un terphényle de formule

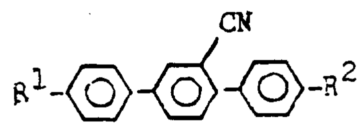

dans laquelle $R^1$ et $R^2$ représentent chacun, indépendamment, un groupe n-alkyle ou n-alcoxy en $C_3$ à $C_{12}$.

**12.** Matière à propriétés de cristaux liquides selon la revendication 10, caractérisée en ce qu'au moins l'un desdits composés est un terphényle de formule

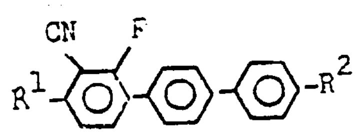

dans laquelle $R^1$ et $R^2$ représentent chacun, indépendamment, un groupe n-alkyle ou n-alcoxy en $C_3$ à $C_{12}$.

**13.** Matière à propriétés de cristaux liquides selon la revendication 10, caractérisée en ce qu'au moins l'un desdits composés est un terphényle ayant une formule choisie parmi les deux ci-après :

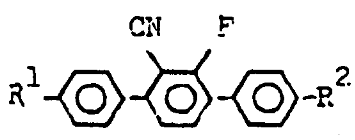

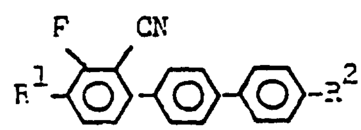

formules dans lesquelles $R^1$ et $R^2$ représentent chacun, indépendamment, un groupe n-alkyle ou n-alcoxy en $C_3$ à $C_{12}$.

**14.** Matière à propriétés de cristaux liquides selon l'une quelconque des revendications 9 à 13, caractérisée en ce que la matière contient en outre un ou plusieurs terphényles fluorés de formule générale

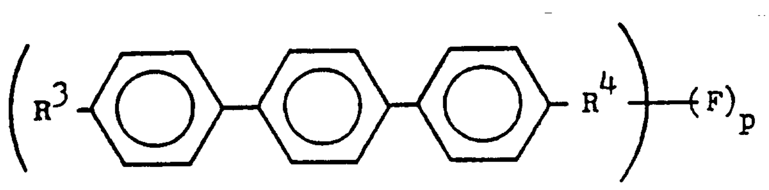

dans laquelle $R^3$ et $R^4$ sont choisis chacun, indépendamment, parmi un atome d'hydrogène, un groupe alkyle et alcoxy contenant chacun 1 à 12 atomes de carbone ; p peut valoir 1 ou 2 et le ou les substituants fluoro peuvent occuper l'une quelconque des positions latérales disponibles pour une substitution.

**15.** Matière à propriétés de cristaux liquides selon la revendication 14, caractérisée en ce que le terphényle fluoré a une formule choisie parmi une ou plusieurs des suivantes :

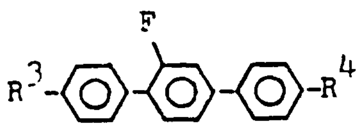 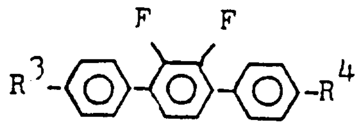 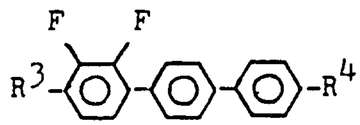

**16.** Matière à propriétés de cristaux liquides selon l'une quelconque des revendications 9 à 13, caractérisée en ce que le mélange contient en outre un ou plusieurs composés de formule

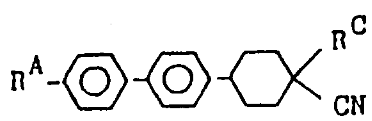

dans laquelle $R^A$ et $R^C$ contiennent chacun, indépendamment, 1 à 12 atomes de carbone ; $R^A$ représente un groupe n-alkyle ou n-alcoxy ; et $R^C$ représente un groupe n-alkyle.

**17.** Matière à propriétés de cristaux liquides selon la revendication 9 ou la revendication 10, caractérisée en ce que le mélange contient en outre un ou plusieurs composés optiquement actifs, de formule

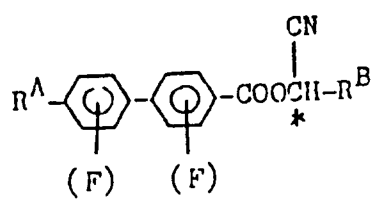

dans laquelle $R^A$ représente un groupe n-alkyle ou n-alcoxy en $C_5$ à $C_{12}$ ; (F) indique que le noyau phényle peut porter un substituant fluoro ; et $R^B$ représente un groupe n-alkyle en $C_1$ à $C_4$, cycloalkyle, ou un groupe alkyle ramifié de formule

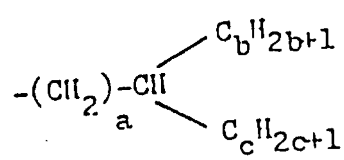

dans laquelle a est nul ou est un nombre entier valant 1 à 6 et chacun des indices b et c représente un nombre entier valant 1 à 6.

**18.** Utilisation d'un dispositif électro-optique à cristaux liquides, sous la forme de deux substrats sensiblement parallèles, dont l'un au moins est optiquement transparent, et comportant, sur leurs surfaces en regard, des électrodes en une matière à propriétés de cristaux liquides, telle que revendiquée à la revendication 9 ou 10.

# Fig.1.

Routes A1 and A2

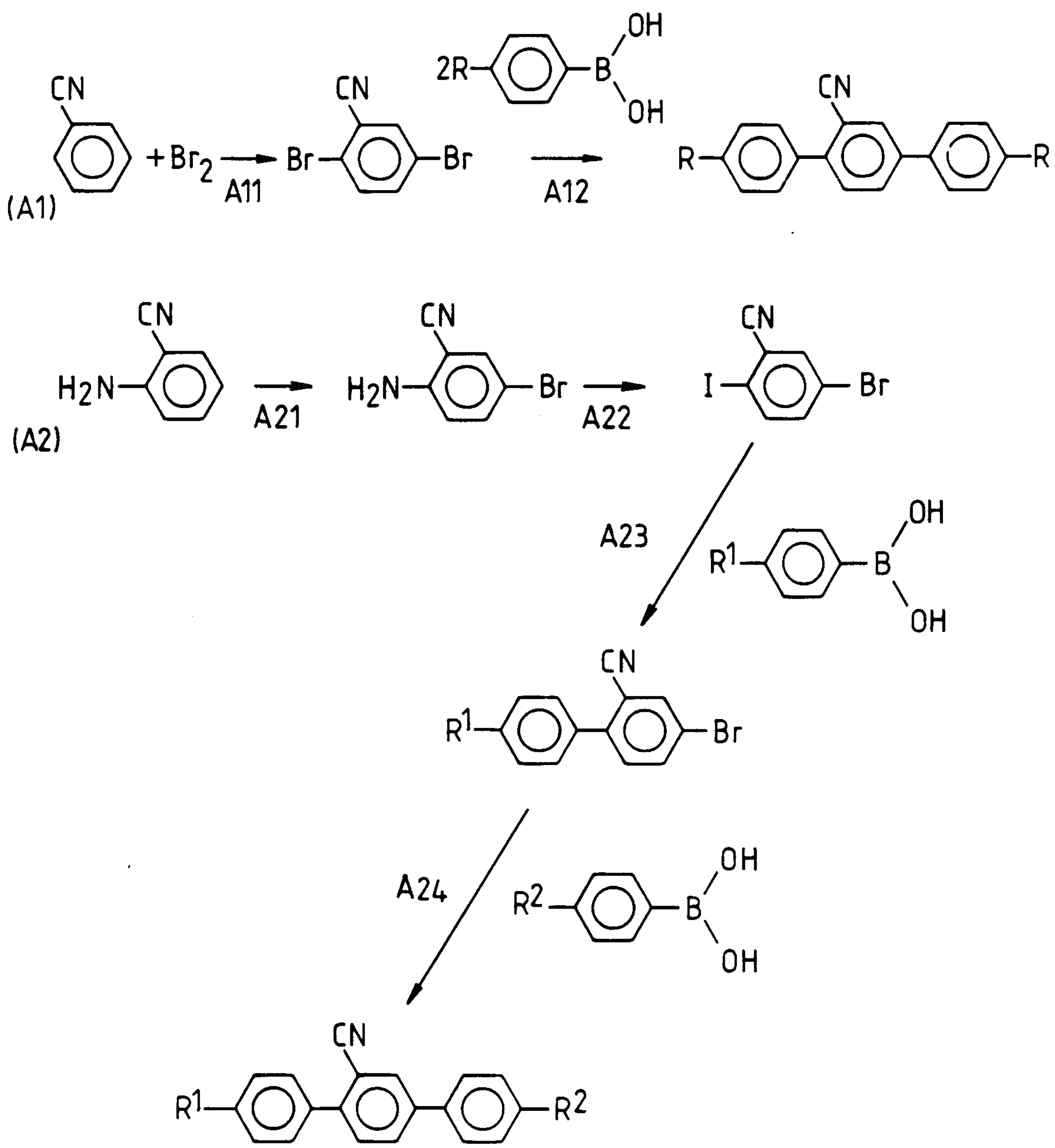


$R, R^1, R^2$ = alkyl or alkoxy

*Fig.2.*

Routes B1 and B2

(B1) 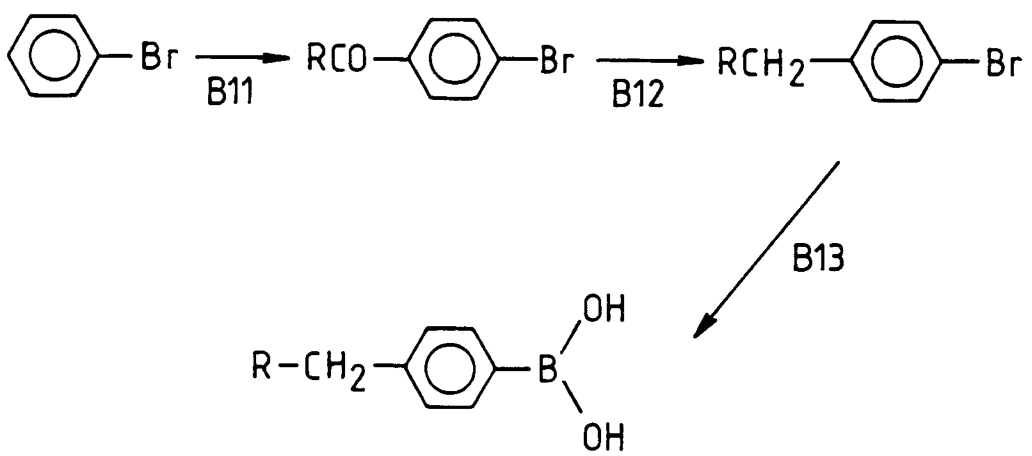


(B2) 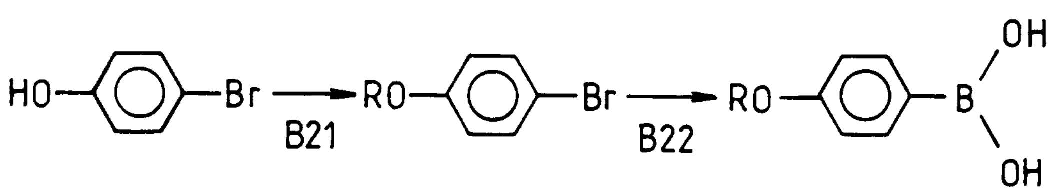


R = alkyl

Route C1

*Fig.3.*

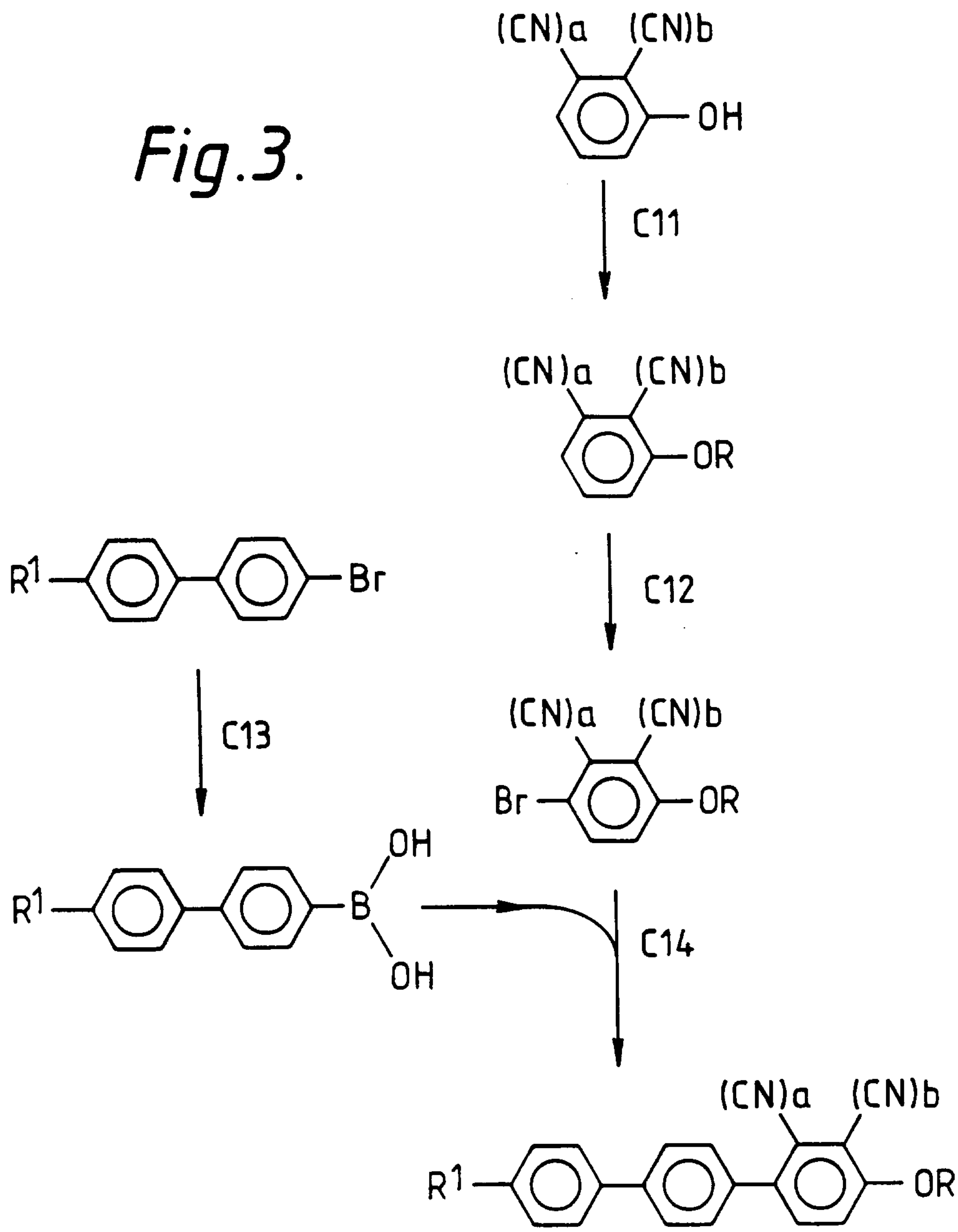


$R^1$= alkyl or alkoxy

R = alkyl

one of a or b = 1
" " " " " = 0

*Fig.4.* Route C2

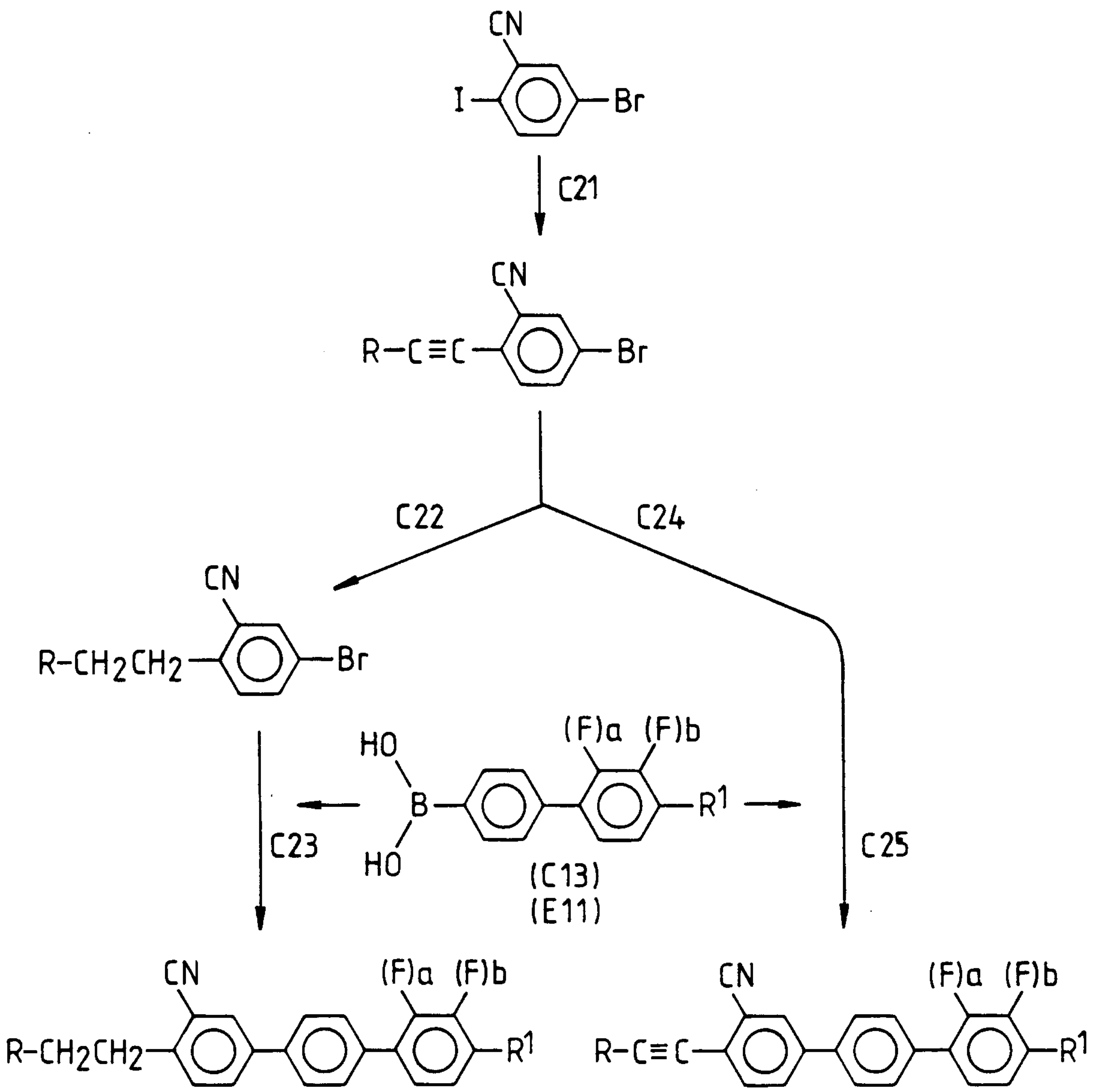


$R^1$ = alkyl or alkoxy

R = alkyl

a = 0 or 1, b = 0 or 1, (a + b) = 0 or 1

*Fig.5.*

Routes D1, D2

(D1)

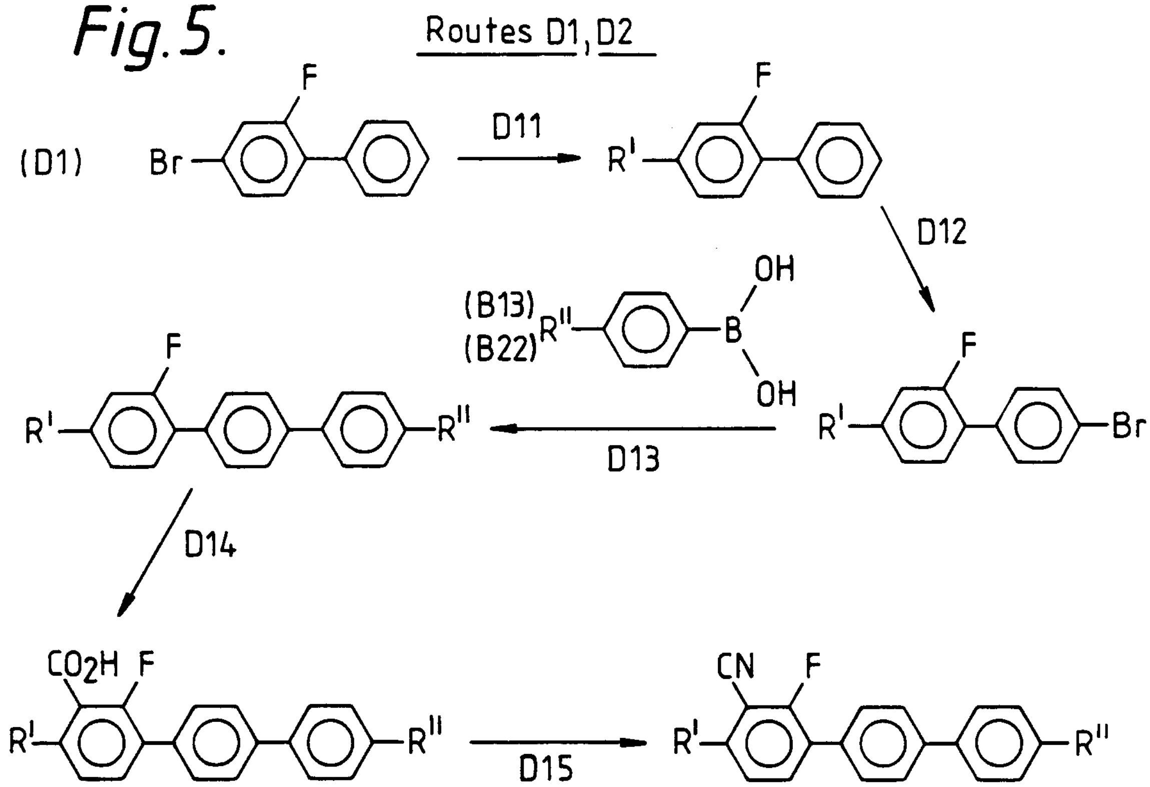


$R^{I}$ = alkyl, alkoxy

(D2)

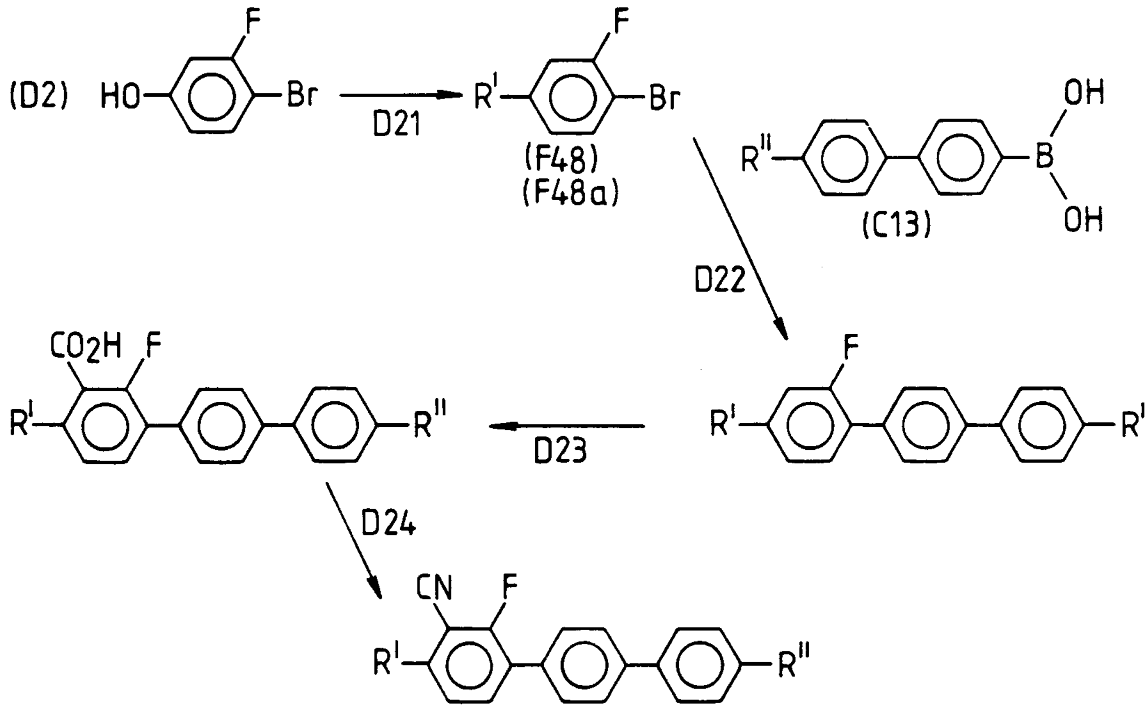


$R^{I}$ = alkyl, alkoxy, alkynyl, $R^{II}$ = alkyl, alkoxy

*Fig. 6.*

Routes D3, D4

(D3)

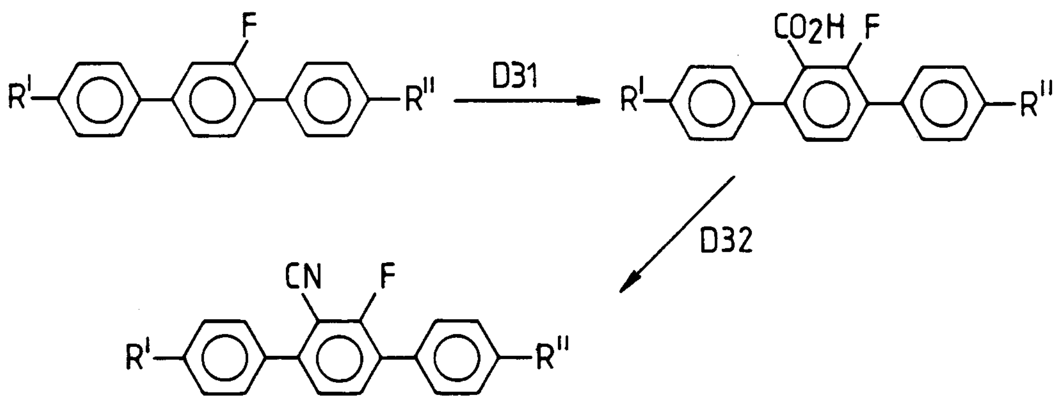


$R^{I}$ — $R^{II}$ = alkyl, alkoxy

(D4)

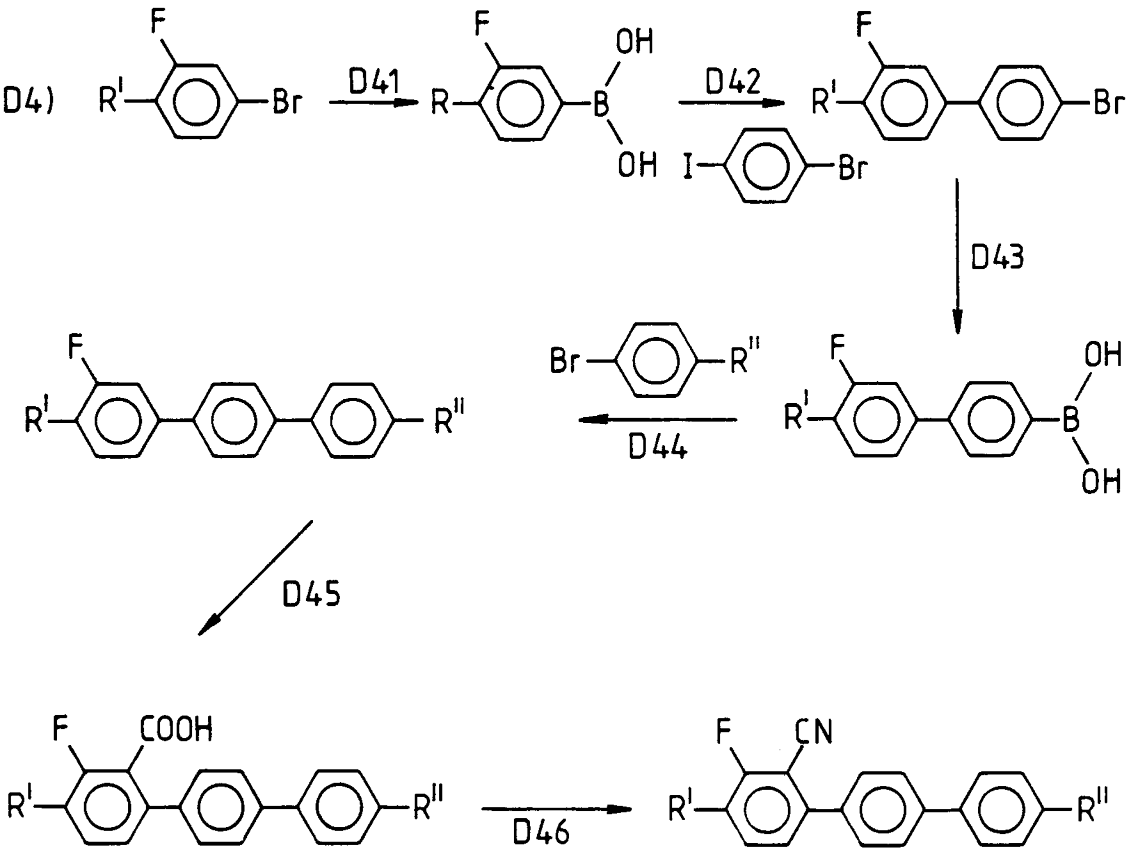


$R^{I}$, $R^{II}$ = alkyl, alkoxy

*Fig. 7.* Route E1

(D12, D43)

(C12)
(C21)

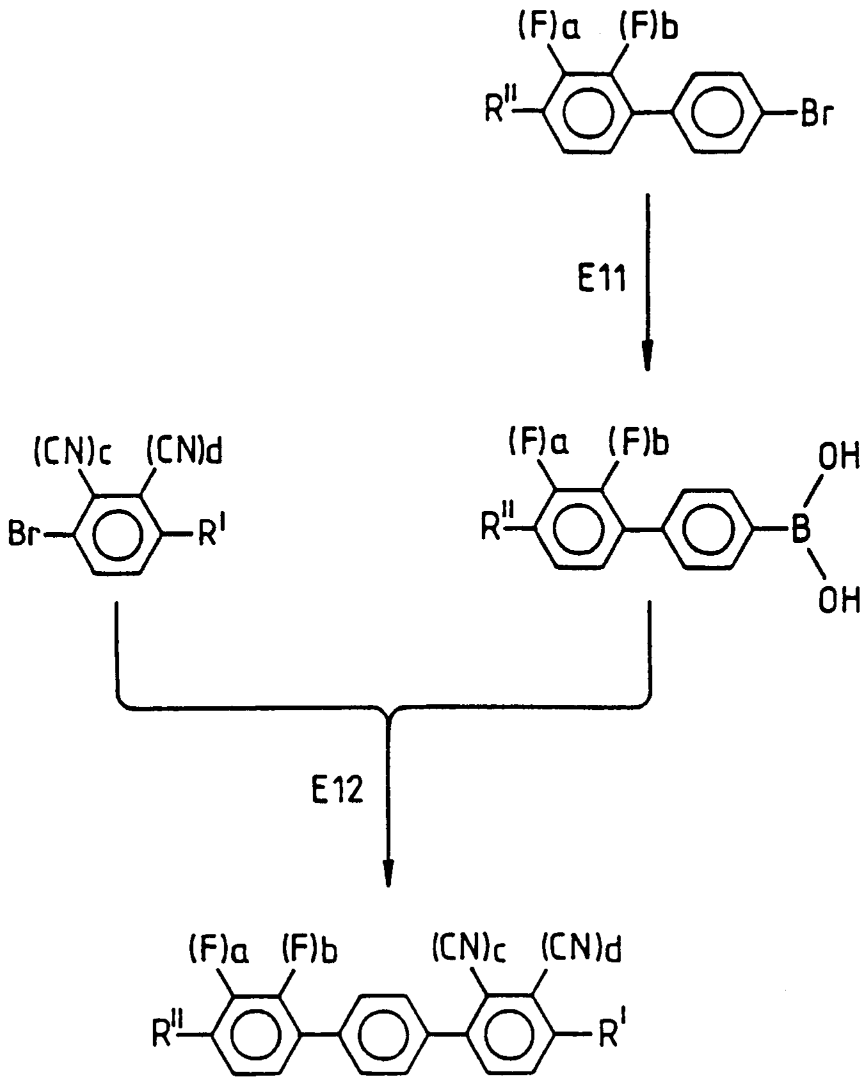


$R^{I}$ = alkyl, alkoxy, alkynyl

$R^{II}$ = alkyl, alkoxy

One of a, b = 1, the other = 0

One of c, d = 1, the other = 0

*Fig.8.* Route E2

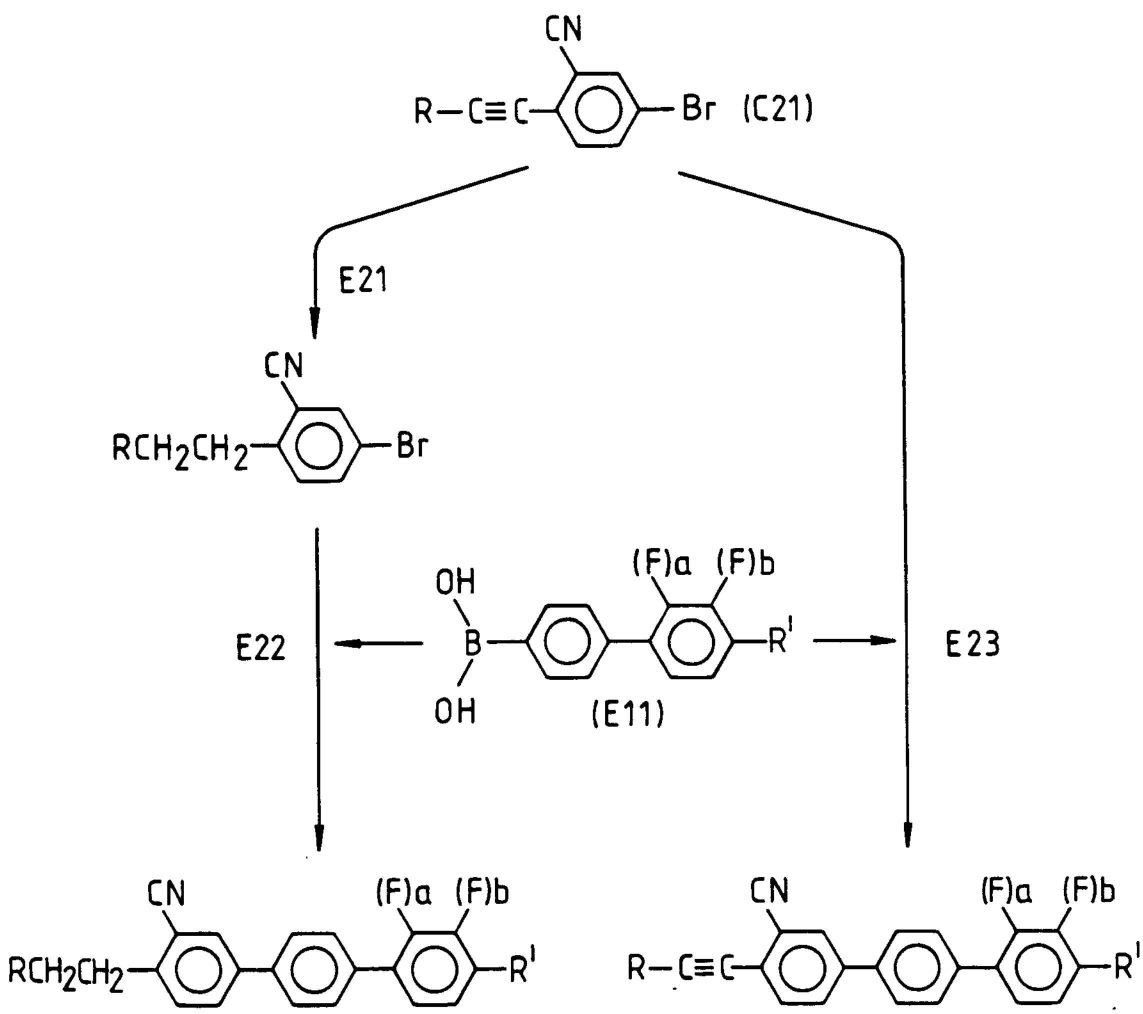


R = alkyl

R' = alkoxy, alkyl

One of a,b=1 the other = 0

*Fig. 9.*

Routes F1, F2

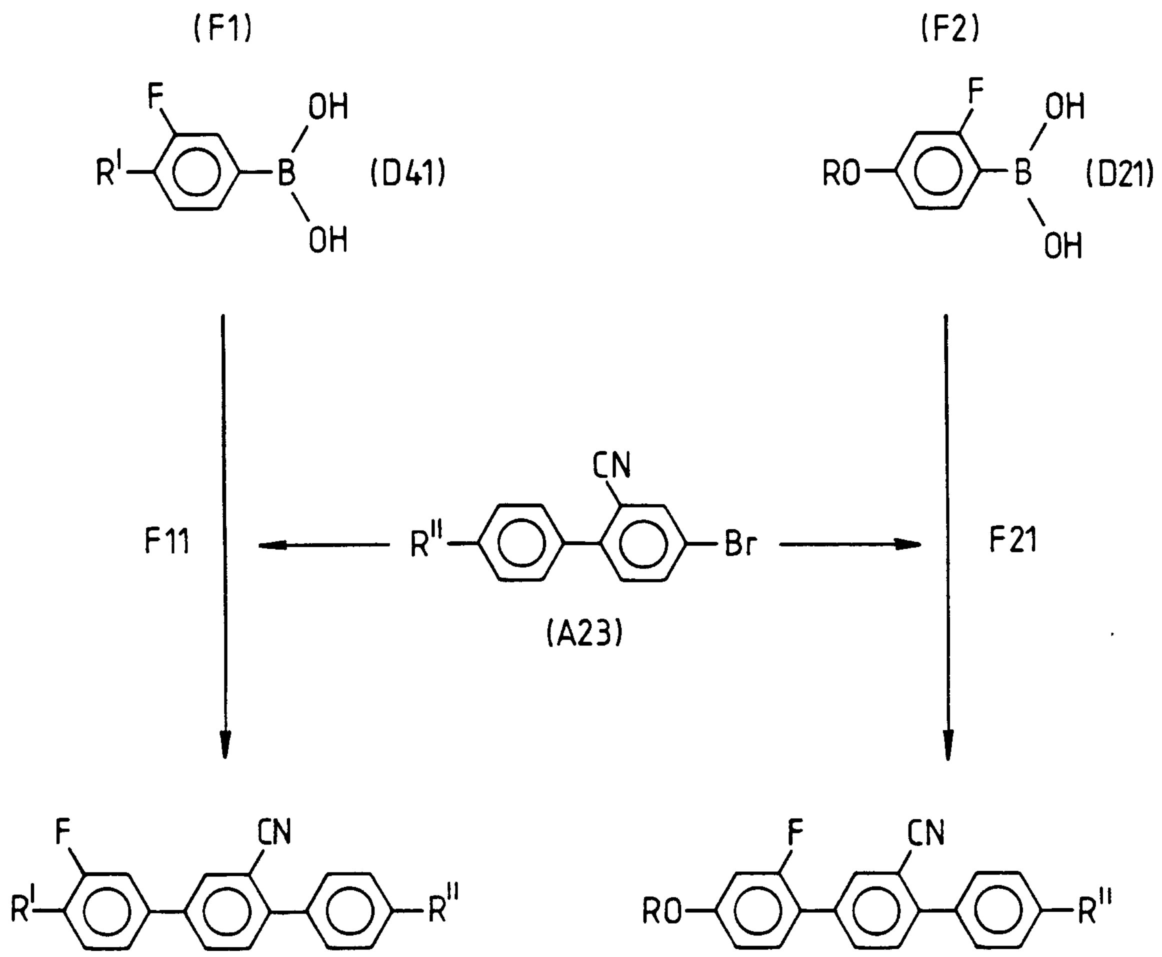


R' = alkyl, alkoxy

R = alkyl

# Fig. 10.

Route F3

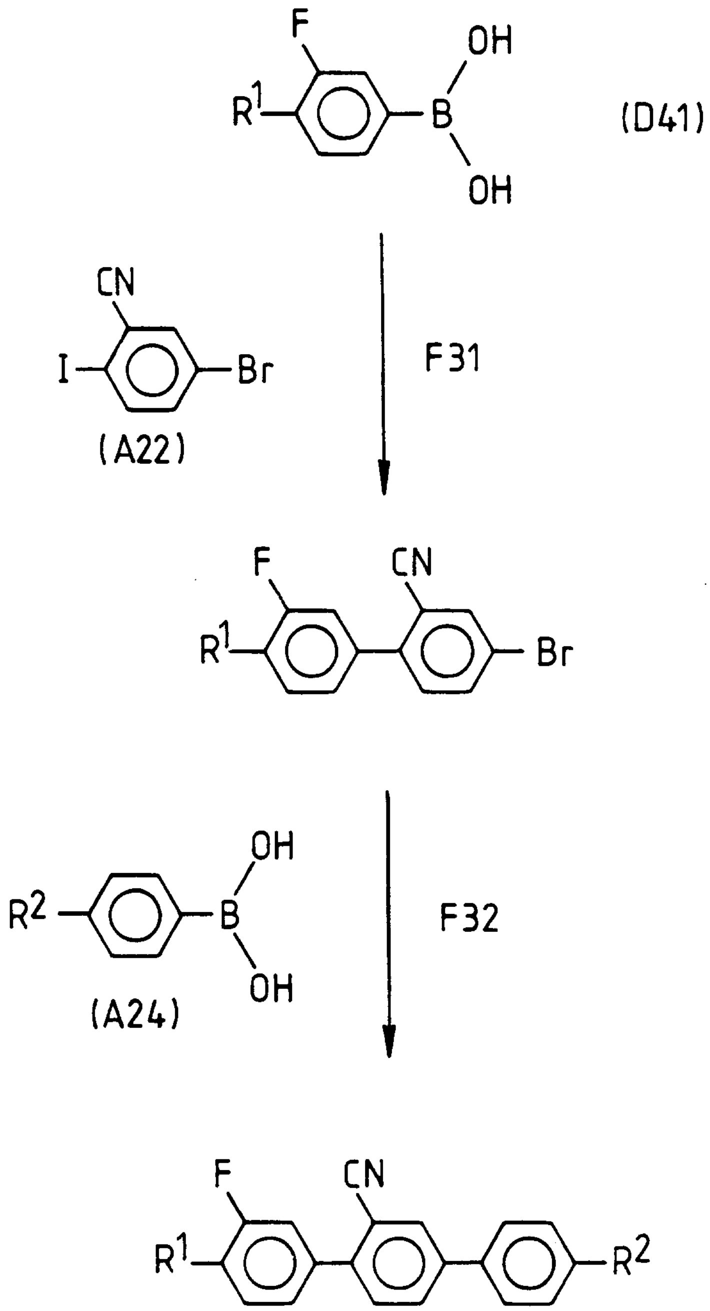


(D41)

(A22)

(A24)

$R^1, R^2$ = alkyl, alkoxy

Route F4

*Fig.11.*

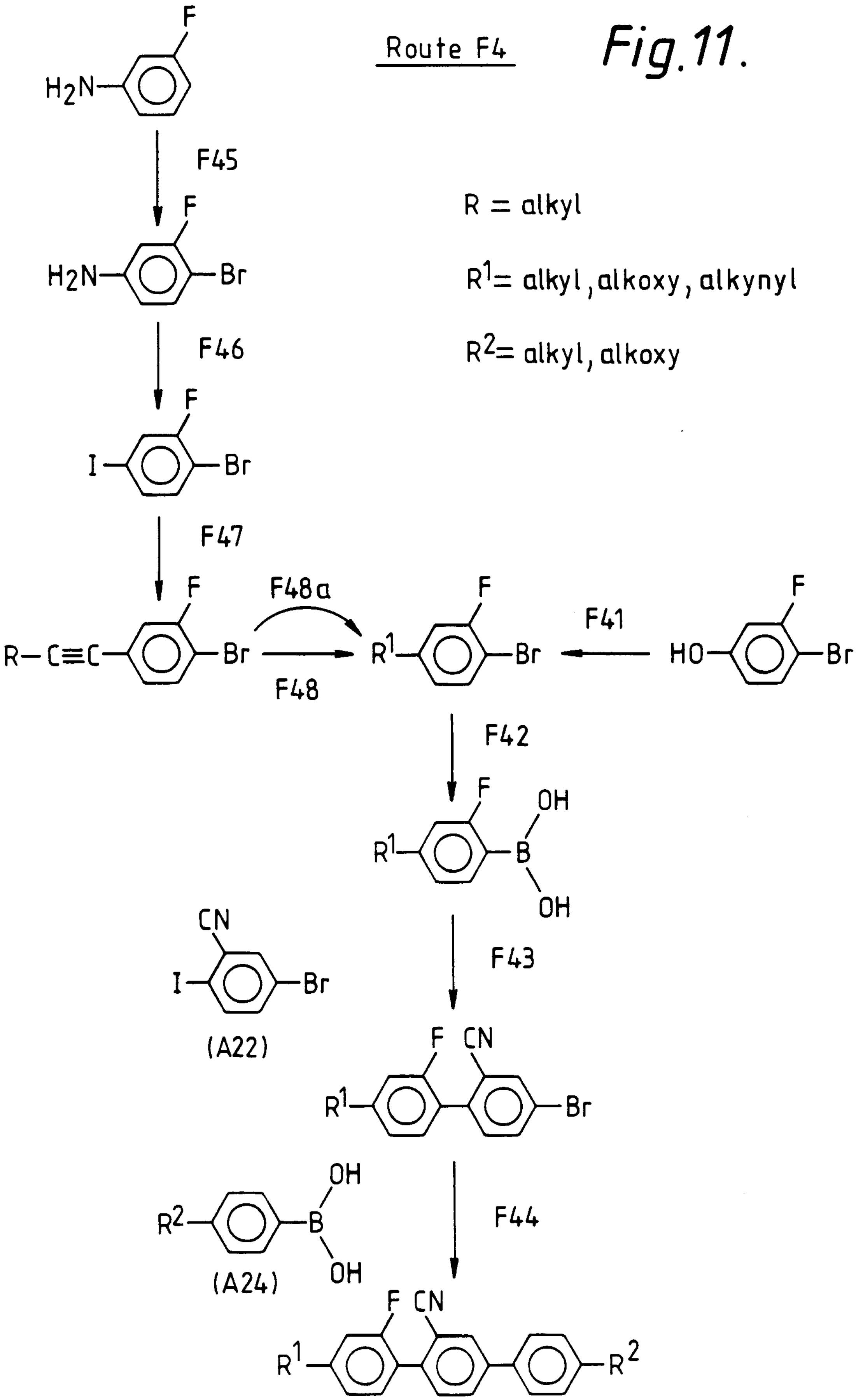


R = alkyl

$R^1$= alkyl, alkoxy, alkynyl

$R^2$= alkyl, alkoxy

(A22)

(A24)

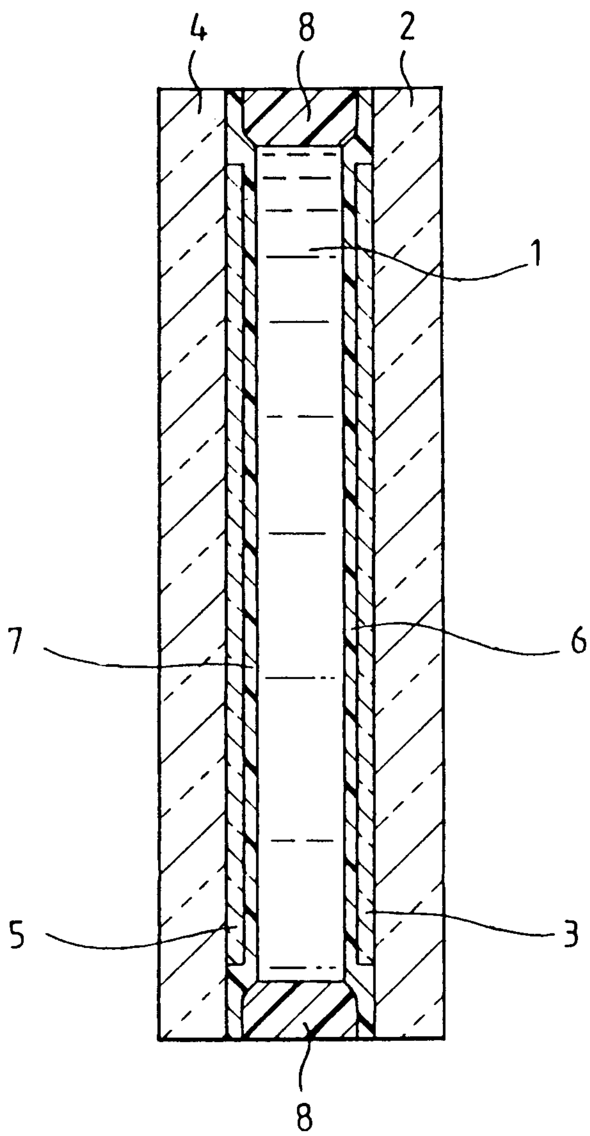


Fig.12.